(19) Europäisches Patentamt
European Patent Office
Office européen des brevets

(11) EP 0 401 623 B1

(12) EUROPÄISCHE PATENTSCHRIFT

(45) Veröffentlichungstag und Bekanntmachung des Hinweises auf die Patenterteilung:
**26.01.2000 Patentblatt 2000/04**

(21) Anmeldenummer: **90110023.0**

(22) Anmeldetag: **26.05.1990**

(51) Int. Cl.$^7$: **C07D 401/04**, C07D 471/04, C07D 207/09, C07D 207/14, A61K 31/47, A61K 31/435 // (C07D401/04, 215:00, 207:00), (C07D471/04, 221:00, 221:00)

(54) **Verfahren zur Herstellung von 7-(3-Amino- sowie 3-Aminomethyl-1-pyrrolidinyl)-3-chinoloncarbonsäuren sowie -naphthyridoncarbonsäuren**

Process for the preparation of 7-(3-amino- and 3-aminomethyl-1-pyrrolidinyl)-3-quinolinonecarboxylic and -naphthyridinonecarboxylic acids

Procédé pour la préparation d'acides 7-(3-amino- et 3-aminométhyl-1-pyrrolidinyl)-3-quinolinonecarboxyliques et -naphtyridinecarboxyliques

(84) Benannte Vertragsstaaten:
**AT BE CH DE DK ES FR GB GR IT LI LU NL SE**

(30) Priorität: **07.06.1989 DE 3918544**

(43) Veröffentlichungstag der Anmeldung:
**12.12.1990 Patentblatt 1990/50**

(73) Patentinhaber: **BAYER AG**
**51368 Leverkusen (DE)**

(72) Erfinder:
- **Petersen, Uwe, Dr.**
  **D-5090 Leverkusen 1 (DE)**
- **Krebs, Andreas, Dr.**
  **D-5068 Odenthal (DE)**
- **Schenke, Thomas, Dr.**
  **D-5060 Bergisch-Gladbach 2 (DE)**
- **Grohe, Klaus, Dr.**
  **D-5068 Odenthal (DE)**
- **Schriewer, Michael, Dr.**
  **D-5068 Odenthal (DE)**
- **Haller, Ingo, Dr.**
  **D-5600 Wuppertal 1 (DE)**
- **Metzger, Karl Georg, Dr.**
  **D-5600 Wuppertal 1 (DE)**
- **Endermann, Rainer, Dr.**
  **D-5600 Wuppertal 1 (DE)**
- **Zeiler, Hans-Joachim, Dr.**
  **D-5620 Velbert (DE)**

(56) Entgegenhaltungen:
**EP-A- 0 132 845** **EP-A- 0 183 129**
**EP-A- 0 191 451** **EP-A- 0 195 316**
**EP-A- 0 208 210** **EP-A- 0 259 804**
**DE-A- 2 739 313** **US-A- 4 762 845**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7/3.6

## Beschreibung

Die vorliegende Erfindung betrifft ein Verfahren zur Herstellung von 7-(3-Amino- sowie 3-Aminomethyl-1-pyrrolidinyl)-3-chinoloncarbonsäuren sowie -naphthyridoncarbonsäuren, welche als antibakterielle Mittel bekanntgeworden sind und durch Umsetzung von 7-Halogen-3-chinoloncarbonsäuren bzw. -naphthyridoncarbonsäuren mit 3-Aminopyrrolidin bzw. 3-Aminomethylpyrrolidin in freier Form oder in Form der 3-Acylamino- bzw. 3-Acylaminomethylpyrrolidine und anschließende Abspaltung der Acylreste hergestellt werden. Umsetzungen dieser Art sind beispielsweise in folgenden Patentanmeldungen beschrieben worden:

Europäische Patentanmeldungen 153 163, 183 129, 195 316, 198 678, 200 307, 207 497, 236 673; US-Patentanmeldung 4 550 104; japanische Patentanmeldung 63 166 876.

Diese Verfahren weisen jedoch verschiedene Nachteile auf:

1. Werden 3-Amino- bzw. 3-Aminomethylpyrrolidine eingesetzt, in denen die Aminogruppe nicht durch einen Acylrest geschützt wird, dann läuft die Umsetzung mit 7-Halogenchinoloncarbonsäuren nicht selektiv im gewünschten Sinne unter Substitution am Pyrrolidin-Stickstoff ab, sondern es treten durch das Vorliegen der freien Aminogruppe auch Nebenprodukte auf, die durch aufwendige Reinigung entfernt werden müßten.
2. Werden 3-Acylamino- bzw. 3-Acylaminomethylpyrrolidine eingesetzt, in denen als Acylrest üblicherweise Acetyl oder tert.-Butoxycarbonyl verwendet wird, so verlaufen die Umsetzungen zu den 7-(3-Acylamino- bzw. 3-Acylaminomethyl-1-pyrrolidinyl)-3-chinoloncarbonsäuren selektiv am Pyrrolidin-Stickstoff. Zur Herstellung der freien 7-(3-Amino-bzw. 3-Aminomethyl-1-pyrrolidinyl)-3-chinoloncarbonsäuren ist die nachträgliche Abspaltung der Schutzgruppe durch Behandeln mit wäßriger Natronlauge in der Hitze bzw. mit Salzsäure oder Trifluoressigsäure erforderlich. Hierdurch können wiederum ungewünschte Nebenreaktionen auftreten: z.B. können die hochwirksamen 8-Brom- bzw. 8-Chlor-3-chinoloncarbonsäuren bei der Deblockierung mit Salzsäure zum Teil das Halogenatom in 8-Stellung verlieren.

Um diese Nachteile zu vermeiden, war die Entwicklung eines Verfahrens erforderlich, daß es gestattet, 7-(3-Amino- bzw. 3-Aminomethyl-1-pyrrolidinyl)-3-chinoloncarbonsäuren in hoher Reinheit auf einfachem Wege herzustellen.

Es wurde gefunden, daß man die Verbindungen der Formel (I), die als Enantiomeren- oder Diastereomerengemische oder in enantiomeren- oder diastereomerenreiner Form vorliegen können,

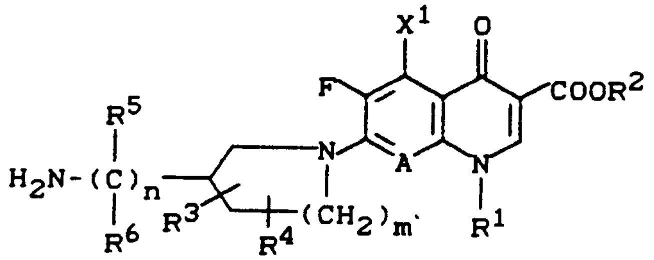

(I)

und in welcher

| | |
|---|---|
| $X^1$ | für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen oder Alkyl mit 1 bis 4 Kohlenstoffatomen, |
| $R^1$ | für geradkettiges oder verzweigtes Alkyl mit 1 bis 5 Kohlenstoffatomen, das gegebenenfalls durch 1 bis 3 Halogenatome substituiert sein kann, sowie Alkenyl mit 2 bis 4 Kohlenstoffatomen, Cycloalkyl mit 3 bis 6 Kohlenstoffatomen, 2-Hydroxyethyl, Methoxy, Amino, Methylamino, Ethylamino, Dimethylamino oder gegebenenfalls durch 1 oder 2 Fluoratome substituiertes Phenyl, |
| $R^2$ | für Wasserstoff, Alkyl mit 1 bis 4 Kohlenstoffatomen oder (5-Methyl-2-oxo-1,3-dioxol-4-yl)-methyl, |
| $R^3$ | für Wasserstoff, $C_1$-$C_3$-Alkyl, Hydroxy, $C_1$-$C_3$-Alkoxy, $C_1$-$C_3$-Alkylthio, Halogen, |
| $R^4$ | für Wasserstoff oder Methyl steht, |
| $R^5$ und $R^6$ | gleich oder verschieden sind und für Wasserstoff oder Methyl stehen |
| m | für 0, 1 oder 2, |
| n | für 0 oder 1 und |
| A | für N oder C-$R^7$ steht, worin |

$R^7$ für H, Halogen, Methyl, Cyano, Nitro, Hydroxy oder Methoxy steht oder auch gemeinsam mit $R^1$ eine Brücke der Struktur

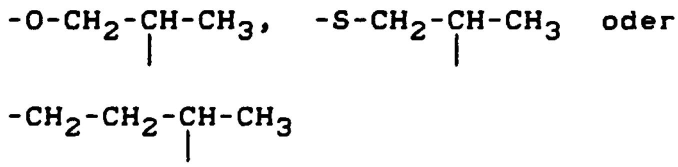

bilden kann, welche R- oder S-Konfiguration besitzen kann,

erhält, indem man Verbindungen der Formel (II)

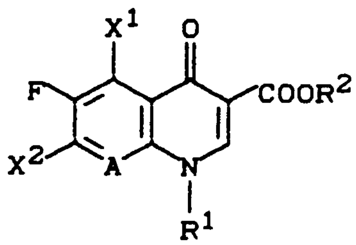

(II),

in welcher

$X^1$, $R^1$, $R^2$ und A die oben angegebene Bedeutung haben und
$X^2$ für Halogen steht,

mit Verbindungen der Formel (III)

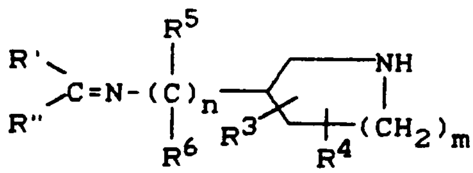

(III),

in welcher

$R^3$, $R^4$, $R^5$, $R^6$, m und n die oben angegebene Bedeutung haben und

R′ für Wasserstoff, geradkettiges oder verzweigtes Alkyl mit 1 bis 10 Kohlenstoffatomen oder Phenyl, das gegebenenfalls durch $C_1$-$C_4$-Alkyl, $C_1$-$C_4$-Alkoxy, $C_1$-$C_2$-Alkylthio, Hydroxy, Nitro, Halogen, Carboxy, $C_1$-$C_4$-Alkoxycarbonyl, Cyano oder Phenyl ein- bis fünffach, gleich oder verschieden substituiert sein kann oder Naphthyl und

R" für Wasserstoff, geradkettiges oder verzweigtes Alkyl mit 1 bis 5 Kohlenstoffatomen oder Phenyl steht, wobei R' und R" auch gemeinsam mit dem Kohlenstoffatom, an welchem sie gebunden sind, einen gegebenenfalls durch Methyl oder Ethyl ein- oder mehrfach substituierten 5- oder 6-gliedrigen aliphatischen Ring bilden können,

gegebenenfalls in Gegenwart eines säurebindenden Mittels zu Verbindungen der Formel (IV)

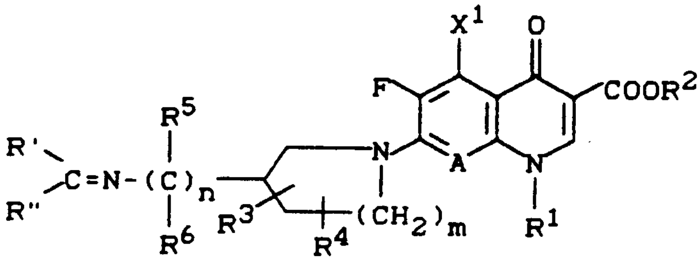

(IV),

in welcher

$X^1$, A, $R^1$, $R^2$, $R^3$, $R^4$, $R^5$, $R^6$, R', R", m und n die oben angegebene Bedeutung haben, umsetzt und anschließend die Aminoschutzgruppe abspaltet.

Bevorzugt werden für das erfindungsgemäße Verfahren Verbindungen der Formel (II)

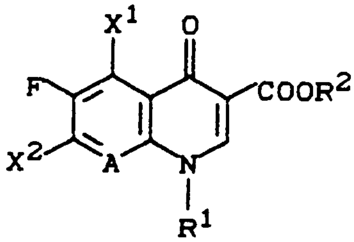

(II),

in welcher

- $X^1$ für Wasserstoff, Amino, Hydroxy, Alkoxy mit 1 oder 2 Kohlenstoffatomen, Fluor, Chlor, Brom, Alkyl mit 1 bis 3 Kohlenstoffatomen,
- $X^2$ für Fluor oder Chlor,
- $R^1$ für geradkettiges oder verzweigte Alkyl mit 1 bis 4 Kohlenstoffatomen, das gegebenenfalls durch 1 bis 3 Fluoratome substituiert sein kann, sowie Alkenyl mit 2 bis 3 Kohlenstoffatomen, Cycloalkyl mit 3 oder 4 Kohlenstoffatomen, 2-Hydroxyethyl, 2-Fluorethyl, Methylamino, Ethylamino, Dimethylamino, gegebenenfalls durch 1 oder 2 Fluoratome substituiertes Phenyl,
- $R^2$ für Wasserstoff, Alkyl mit 1 bis 3 Kohlenstoffatomen und
- A für N oder C-$R^7$ steht, worin

  $R^7$ für H, Fluor, Chlor, Brom, Methyl, Hydroxy oder Methoxy steht oder auch gemeinsam mit $R^1$ eine Brücke der Struktur

$$-O-CH_2-\underset{|}{C}H-CH_3$$

  bilden kann,

sowie Verbindungen der Formel (III)

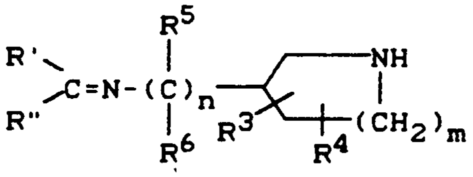

(III),

in welcher

$R^3$ für Wasserstoff, Methyl, Ethyl, Hydroxy, Methoxy, Ethoxy, Methylthio, Ethylthio, Chlor oder Fluor,
$R^4$ für Wasserstoff oder Methyl,
$R^5$ für Wasserstoff,
$R^6$ für Wasserstoff,
m für 0 oder 1,
n für 0 oder 1,
R' für Wasserstoff, geradkettiges oder verzweigtes Alkyl mit 1 bis 6 Kohlenstoffatomen oder Phenyl, das gegebenenfalls durch Methyl, Methoxy, Ethoxy, Hydroxy, Nitro, Fluor, Chlor, Carboxy, $C_1$-$C_3$-Alkoxycarbonyl oder Cyano ein- bis fünffach, gleich oder verschieden substituiert sein kann, und
R'' für Wasserstoff, geradkettiges oder verzweigtes Alkyl mit 1 bis 4 Kohlenstoffatomen oder Phenyl steht, wobei R' und R'' auch gemeinsam mit dem Kohlenstoffatom, an welchem sie gebunden sind, einen 5- oder 6-gliedrigen aliphatischen Ring bilden können.

Besonders bevorzugt werden für das erfindungsgemäße Verfahren Verbindungen der Formel (II)

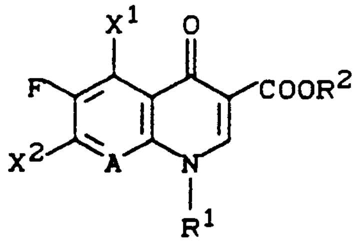

(II),

in welcher

$X^1$ für Wasserstoff, Amino, Methoxy, Fluor, Chlor oder Methyl,
$X^2$ für Fluor oder Chlor,
$R^1$ für Methyl, Ethyl, tert.-Butyl, Vinyl, Cyclopropyl, 2-Fluorethyl, 4-Fluorphenyl oder 2,4-Difluorphenyl,
$R^2$ für Wasserstoff, Methyl oder Ethyl,
A für N oder C-$R^7$ steht, worin

$R^7$ für Wasserstoff, Fluor, Chlor, Methyl oder Methoxy steht oder auch gemeinsam mit $R^1$ eine Brücke der Struktur

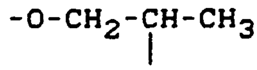

bilden kann,

sowie Verbindungen der Formel (III)

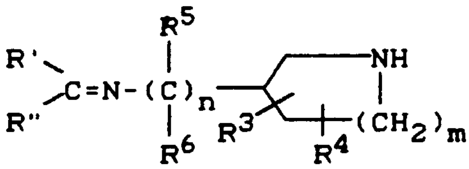

(III),

in welcher

$R^3$ für Wasserstoff, Methyl, Hydroxy, Methoxy, Methylthio, Ethylthio oder Fluor,
$R^4$ für Wasserstoff oder Methyl,
$R^5$ für Wasserstoff,
$R^6$ für Wasserstoff,
m für 0 oder 1,
n für 0 oder 1,
R' für Wasserstoff, geradkettiges oder verzweigtes Alkyl mit 1 bis 5 Kohlenstoffatomen oder Phenyl, das gegebenenfalls durch Methyl, Methoxy, Hydroxy, Nitro, Fluor, Chlor, Carboxy oder $C_1$-$C_2$-Alkoxycarbonyl ein- bis vierfach, gleich oder verschieden substituiert sein kann, und
R" für Wasserstoff, geradkettiges oder verzweigtes Alkyl mit 1 bis 3 Kohlenstoffatomen oder Phenyl steht, wobei R' und R" auch gemeinsam mit dem Kohlenstoffatom, an welchem sie gebunden sind, einen 5- oder 6-gliedrigen aliphatischen Ring bilden können.

Die Verbindungen der Formeln (III) und (IV) sind ebenfalls Gegenstand der Erfindung, wobei für Verbindungen der Formel (IV) solche ausgenommen sind, in denen gleichzeitig R" für H, n für 0, m für 1, $R^3$ und $R^4$ für H, $X^1$ für H, A für CH, $R^2$ für H oder eine Carboxy schützende Gruppe, $R^1$ für $C_1$-$C_5$-Alkyl, $C_3$-$C_6$-Cycloalkyl gegebenenfalls durch 1 oder 2 Fluoratome substituiertes Phenyl und R' für Phenyl oder substituiertes Phenyl steht.

Die als Ausgangsstoffe verwendeten Verbindungen der Formel (II) sind bekannt oder können nach bekannten Methoden hergestellt werden. Als Beispiele seien genannt:

7-Chlor-1-cyclopropyl-6-fluor-1,4-dihydro-4-oxo-3-chinolincarbonsäure (Deutsche Patentanmeldung 3 142 854),
1-Cyclopropyl-6,7-difluor-1,4-dihydro-4-oxo-3-chinolincarbonsäure (Europäische Patentanmeldung 113 091),
8-Chlor-1-cyclopropyl-6,7-difluor-1,4-dihydro-4-oxo-3-chinolincarbonsäure (Deutsche Patentanmeldung 3 420 743),
1-Cyclopropyl-6,7,8-trifluor-1,4-dihydro-4-oxo-3-chinolin-carbonsäure (Deutsche Patentanmeldung 3 318 145),
1-Cyclopropyl-6,7-difluor-1,4-dihydro-8-methyl-4-oxo-3-chinolincarbonsäure,
6,7-Difluor-1-ethyl-1,4-dihydro-4-oxo-3-chinolincarbonsäure,
7-Chlor-6-fluor-1-ethyl-1,4-dihydro-4-oxo-3-chinolincarbonsäure,
7-Chlor-6-fluor-1,4-dihydro-1-(2-hydroxyethyl )-4-oxo-3-chinolincarbonsäure,
1-Cyclopropyl-7-chlor-6-fluor-1,4-dihydro-8-nitro-4-oxo-3-chinolincarbonsäure,
6,7-Difluor-1-(2-fluorethyl)-1,4-dihydro-4-oxo-3-chinolincarbonsäure,
6,7-Difluor-1,4-dihydro-1-methoxy-4-oxo-3-chinolincarbonsäure,
6,7-Difluor-1,4-dihydro-1-methylamino-4-oxo-3-chinolincarbonsäure,
6,7-Difluor-1,4-dihydro-4-oxo-1-phenyl-3-chinolincarbonsäure,
6,7-Difluor-1-(4-fluor-phenyl)-1,4-dihydro-4-oxo-3-chinolincarbonsäure,
6,7-Difluor-1-(3,4-difluor-phenyl)-1,4-dihydro-4-oxo-3-chinolincarbonsäure,
7-Chlor-1-cyclopropyl-6-fluor-1,4-dihydro-4-oxo-1,8-naphthyridin-3-carbonsäure,
1-Cyclopropyl-6,7,8-trifluor-1,4-dihydro-4-oxo-3-chinolincarbonbonsäureethylester (Deutsche Patentanmeldung 3 318 145),
9,10-Difluor-2,3-dihydro-3-methyl-7-oxo-7H-pyrido-[1,2,3-de][1,4]benzoxacin-6-carbonsäure (Europäische Patentanmeldung 47 005) beziehungsweise deren R- oder S-Form (Deutsche Patentanmeldung 3 543 513),
8,9-Difluor-6,7-dihydro-5-methyl-1-oxo-1H,5H-benzo[i,j]chinolicin-2-carbonsäure,
7-Chlor-6-fluor-1-phenyl-1,4-dihydro-4-oxo-1,8-naphthyridin-3-carbonsäure (Europäische Patentanmeldung 153 580),
7-Chlor-6-fluor-1-(4-fluorphenyl)-1,4-dihydro-4-oxo-1,8-naphthydrin-3-carbonsäure (Europäische Patentanmeldung 153 580),

6,7,8-Trifluor-1,4-dihydro-1-methylamino-4-oxo-3-chinolincarbonsäure (Deutsche Patentanmeldung 3 409 922),
1-Amino-6,7,8-trifluor-1,4-dihydro-4-oxo-3-chinolincarbonsäure (Deutsche Patentanmeldung 3 409 922),
6,7,8-Trifluor-1,4-dihydro-1-dimethylamino-4-oxo-3-chinolincarbonsaure (Deutsche Patentanmeldung 3 409 922),
7-Chlor-6-fluor-1,4-dihydro-8-nitro-4-oxo-1-phenyl-3-chinolincarbonsäure,
7-Chlor-6-fluor-1-(4-fluorphenyl)-1,4-dihydro-8-nitro-4-oxo-3-chinolincarbonsäure,
6,7-Difluor-1-(4-fluorphenyl)-1,4-dihydro-8-methyl-4-oxo-3-chinolincarbonsäure,
6-Chlor-7-fluor-1-(4-fluorphenyl)-1,4-dihydro-4-oxo-3-chinolincarbonsäure (Europäische Patentanmeldung 131 839),
6-Chlor-7-fluor-1-(2,4-difluorphenyl)-1,4-dihydro-4-oxo-3-chinolincarbonsäure (Europäische Patentanmeldung 131 839),
6,7,8-Trifluor-1-(4-fluorphenyl)-1,4-dihydro-4-oxo-3-chinolincarbonsäure (Europäische Patentanmeldung 154 780),
6,7,8-Trifluor-1-(2,4-difluorphenyl)-1,4-dihydro-4-oxo-3-chinolincarbonsäure (Europäische Patentanmeldung 154 780),
6,7,8-Trifluor-1,4-dihydro-4-oxo-1-phenyl-3-chinolincarbonsäure (Europäische Patentanmeldung 154 780),
7-Chlor-1-ethyl-6-fluor-1,4-dihydro-4-oxo-1,8-naphthyridin-3-carbonsäure,
6,7-Difluor-1,4-dihydro-4-oxo-1-vinyl-3-chinolincarbonsäure.
1-tert.-Butyl-7-chlor-6-fluor-1,4-dihydro-4-oxo-1,8-naphthyridin-3-carbonsäure,
1-tert.-Butyl-6,7-difluor-1,4-dihydro-4-oxo-3-chinolincarbonsäure
1-tert.-Butyl-6,7,8-trifluor-1,4-dihydro-4-oxo-3-chinolincarbonsäure,
1-Cyclopropyl-6,7-difluor-1,4-dihydro-5-methyl-4-oxo-3-chinolincarbonsäure und
7-Chlor-1-(2,4-difluorphenyl)-6-fluor-1,4-dihydro-4-oxo-1,8-naphthyridin-3-carbonsäureethylester.

Die Zwischenverbindungen der Formel (III) sind neu. Ihre Herstellung erfolgt durch Kondensation einer Oxoverbindung der Formel (V) mit einer Aminoverbindung der Formel (VI) unter Abspaltung von Wasser.

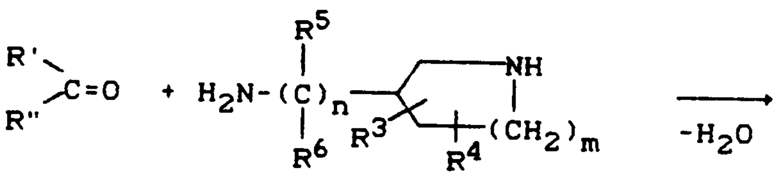


(III)

(V) (VI)

Chirale Aminoverbindungen der Formel (VI) können sowohl als Racemate als auch als enantiomerenreine oder diastereomerenreine Verbindungen eingesetzt werden.

Die Umsetzung von (V) mit (VI) wird entweder ohne Verdünnungsmittel oder in einem Verdünnungsmittel wie beispielsweise Acetonitril, Dimethylformamid, Dimethylsulfoxid, Sulfolan, einem Alkohol wie Methanol, Ethanol, Propanol oder Isopropanol, einem Ether wie Diethylether, Diisopropylether, Glykolmonomethylether, tert.-Butyl-methylether, Dioxan oder Tetrahydrofuran, einem aromatischen oder aliphatischen Kohlenwasserstoff wie Toluol, Xylol, Chlorbenzol, Dichlormethan, Pentan, Hexan oder Cyclohexan oder Pyridin vorgenommen. Ebenso können Gemische dieser Verdünnungsmittel verwendet werden.

Die Umsetzung von (V) mit (VI) wird gewöhnlich ohne weitere Zusätze durchgeführt. Man kann aber auch katalytische Mengen einer anorganischen oder organischen Säure wie Schwefelsäure, Chlorwasserstoffsäure, Benzolsulfonsäure, p-Toluolsulfonsäure oder einem sauren Ionenaustauscher zusetzen. Als wasserbindende Mittel können Natriumsulfat, Magnesiumsulfat, Calciumchlorid oder ein Molekularsieb zugegeben werden. Man kann auch in einer wasserabscheidenden Apparatur arbeiten.

Die Reaktionstemperaturen können in einem größeren Bereich variiert werden. Im allgemeinen arbeitet man zwischen etwa -20°C und 120°C, vorzugsweise zwischen 0°C und 50°C.

Die Umsetzung kann bei Normaldruck, aber auch bei erhöhtem Druck durchgeführt werden. Im allgemeinen arbeitet man bei Drucken zwischen etwa 1 und etwa 10 bar, vorzugsweise bei Normaldruck.

Die Verbindungen der Formel (III) können aus dem Reaktionsgemisch, beispielsweise durch fraktionierte Destillation gewonnen werden.

Man kann die Verbindungen der Formel (III) aber auch ohne Zwischenisolierung vorteilhaft im Eintopfverfahren mit den Verbindungen der Formel (II) zu Verbindungen der Formel (I) umsetzen. Diese In-situ-Herstellung bietet sich

besonders dann an, wenn die Verbindungen der Formel (III) schwierig zu isolieren sind, wie beispielsweise bei der Umsetzung von 3-Aminopyrrolidin mit aromatischen Aldehyden. Andererseits kann die Isolierung des Zwischenproduktes der Formel (III) dann von Vorteil sein, wenn die Aminokomponente (VI) aus einem Reaktionsgemisch heraus isoliert werden muß. Wird z.B. 3-Aminopyrrolidin durch hydrogenolytische Debenzylierung von 1-Benzyl-3-benzylamino-pyrrolidin hergestellt, so kann man die rohe Reaktionslösung ohne Zwischenisolierung des 3-Aminopyrrolidins mit Pivalaldehyd umsetzen und auf einfache Weise das 3-(2,2-Dimethyl-propylidenamino)pyrrolidin isolieren.

Das erfindungsgemäße Verfahren, nämlich der Schutz der Aminoverbindungen der Formel (VI) durch eine Carbonyl-Verbindung der Formel (V) und die anschließende selektive Umsetzung des Reaktionsproduktes (III) mit 7-Halogenchinoloncarbonsäuren der Formel (II) ist insofern als außerordentlich überraschend anzusehen, als es aus der Literatur bekannt ist, daß Aldehyde mit offenkettigen oder cyclischen Diaminen cyclische bzw. bicyclische Aminale bilden [siehe z.B. DE 2 739 313; J. Amer. Chem. Soc. 95, 3362 (1973); J. Org. Chem. 53 420 (1988); Liebigs Ann. Chem. 1977, 956 und Eur. J. Med. Chem. 17, 235 (1982)]. Deshalb können Aldehyde mit Diaminen in metallorganischen Reaktionen geschützt werden [Aust. J. Chem. 26, 1363 (1973) und Tetrahedron 41, 3803 (1985)].

Mit Hinblick auf diese Veröffentlichungen mußte erwartet werden, daß beispielsweise 3-Amino-pyrrolidin mit Aldehyden zu bicyclischen Aminalen reagiert:

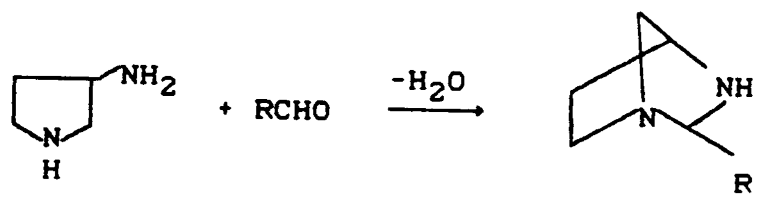


Es war daher zu erwarten, daß die bicyclischen Aminale mit 7-Halogen-4-chinolon-3-carbonsäuren bzw. -naphtyridon-carbonsäuren der Formel (II) eher am sekundären als am tertiären Stickstoffatom reagieren und nach Abspaltung der Schutzgruppe zu 7-(Pyrrolidin-3-yl-amino)chinolon- bzw. -naphthyridon-3-carbonsäuren führen würde, so daß die spezifische Reaktion zu 7-(3-Amino-1-pyrrolidinyl)-4-chinolon-3-carbonsäuren bzw. -naphthyridon-carbonsäuren der Formel (I) überraschend ist.

Werden beispielsweise 1-Cyclopropyl-6,7,8-trifluor-1,4-dihydro-4-oxo-3-chinolincarbonsäure mit 3-(2,2-Dimethylpropylidenamino)-pyrrolidin umgesetzt und das Reaktionsprodukt mit Salzsäure behandelt, dann erhält man 7-(3-Amino-1-pyrrolidinyl)-1-cyclopropyl-6,8-difluor-1,4-dihydro-4-oxo-3-chinolincarbonsäure-Hydrochlorid.

Die Reaktion läßt sich durch folgendes Schema wiedergeben:

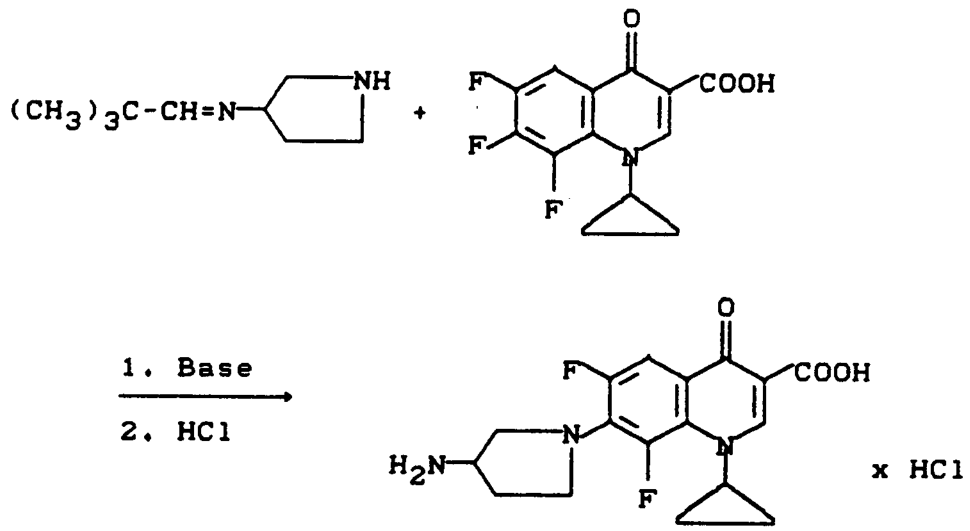


Setzt man beispielsweise in einer Eintopfreaktion das Reaktionsgemisch aus 3-Nitrobenzaldehyd und (S)-3-Aminopyrrolidin mit 8-Chlor-1-cyclopropyl-6,7-difluor-1,4-dihydro-4-oxo-3-chinolincarbonsäure um, dann läßt sich der Reaktionsablauf über das intermediär entstehende 8-Chlor-1-cyclopropyl-6-fluor-1,4-dihydro-7-[(S)-3-(3-nitrobenzylidenamino)-1-pyrrolidinyl]-4-oxo-3-chinolincarbonsäure zum 7-[(S)-3-Amino-1-pyrrolidinyl]-8-chlor-1-cyclopropyl-6-fluor-1,4-dihydro-4-oxo-3-chinolincarbonsäure-Hydrochlorid durch folgendes Formelschema wiedergeben:

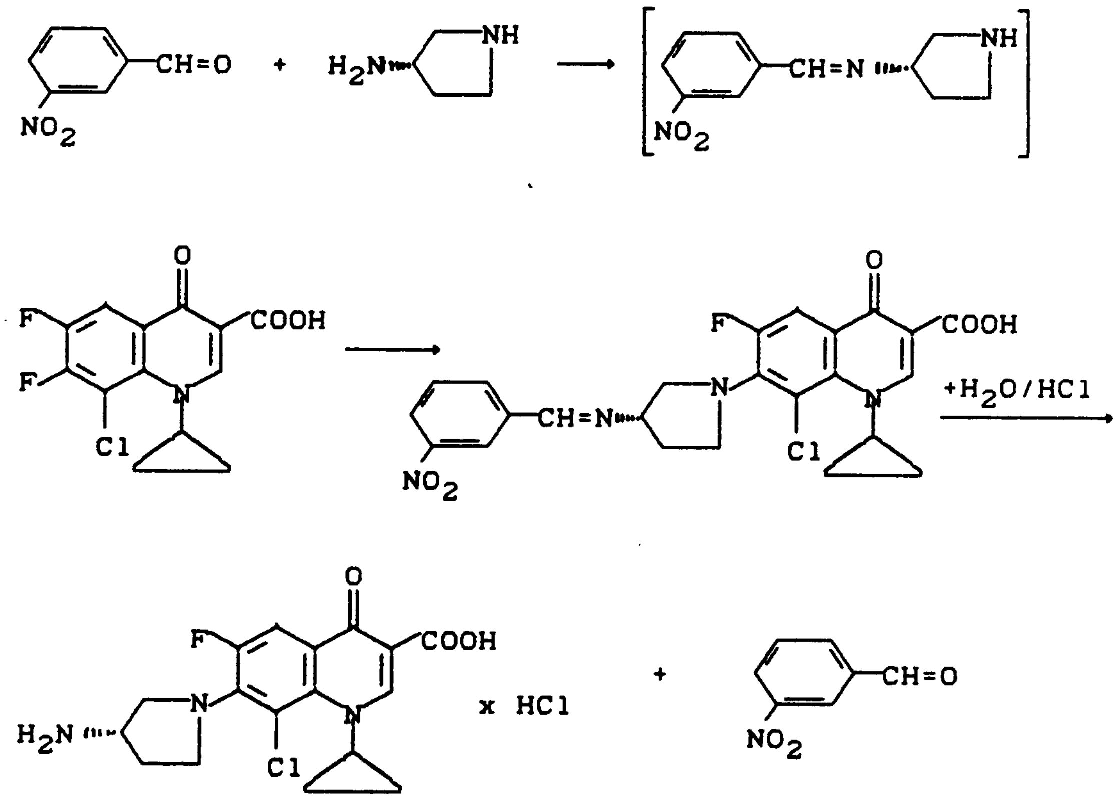


Die Ausgangsverbindungen der Formel (V) sind bekannt. Beispielhaft seien folgende Verbindungen genannt:

Benzaldehyd, 2-Chlorbenzaldehyd, 3-Chlorbenzaldehyd, 4-Chlorbenzaldehyd, 2,4-Dichlorbenzaldehyd, 2,6-Dichlorbenzaldehyd, 2,4,5-Trichlorbenzaldehyd, 2-Nitrobenzaldehyd, 3-Nitrobenzaldehyd, 4-Nitrobenzaldehyd, 4-Chlor-3-nitrobenzaldehyd, 2-Chlor-5-nitrobenzaldehyd, 2-Methylbenzaldehyd, 3-Methylbenzaldehyd, 4-Methylbenzaldehyd, 3-Phenylpropionaldehyd, Zimtaldehyd, 2-, 3- und 4-Hydroxybenzaldehyd, 3,5-Dichlor-2-hydroxybenzaldehyd, 2-Hydroxy-1-naphthaldehyd, 2-Hydroxy-3-methoxy-benzaldehyd, 4-Hydroxy-3-methoxy-benzaldehyd, 2-, 3- und 4-Methoxybenzaldehyd, 2-, 3- und 4-Pyridincarboxaldehyd, 2-Fluorbenzaldehyd, 4-Fluorbenzaldehyd, 3-Fluorbenzaldehyd, 2,4-Difluorbenzaldehyd, 3,5-Difluorbenzaldehyd, Acetaldehyd, Propionaldehyd, Butyraldehyd, 2-Methylbutanal, 2,2-Dimethyl-propanal, Trichloracetaldehyd-Hydrat (Chloralhydrat), Glyoxal, Aceton, Butanon, 3-Methyl-2-butanon, Cyclohexanon, Cyclopentanon.

Anstelle der Oxoverbindungen (V) können auch Derivate verwendet werden, die sich unter den Reaktionsbedingungen wie Oxoverbindungen verhalten, wie z.B. Acetale oder Ketale.

Als Beispiele für Ausgangsverbindungen der Formel (VI) seien folgende Verbindungen genannt, wobei chirale Verbindungen sowohl als Racemate als auch als enantiomerenreine oder diastereomerenreine Stoffe eingesetzt werden können:

R,S-3-Amino-pyrrolidin,
R-3-Amino-pyrrolidin,
S-3-Amino-pyrrolidin,
cis- und trans-3-Amino-4-methoxy-pyrrolidin,
3-Amino-2-methyl-4-methoxy-pyrrolidin,
4-Amino-2-methyl-3-methoxy-pyrrolidin,
cis- und trans-3-Amino-4-methyl-pyrrolidin,
3-Amino-3-methyl-pyrrolidin,

3-Amino-2-methyl-pyrrolidin,
4-Amino-2-methyl-pyrrolidin,
3-Aminomethyl-pyrrolidin,
3-Aminomethyl-3-hydroxy-pyrrolidin,
3-Aminomethyl-3-methoxy-pyrrolidin,
3-Aminomethyl-3-methyl-pyrrolidin,
4-Amino-2-hydroxymethyl-pyrrolidin,
3-(1-Aminoethyl)-pyrrolidin,
3-Amino-azetidin,
3-Aminomethyl-azetidin,
3-Amino-piperidin,
4-Amino-piperidin,
cis- und trans-3-Amino-4-methylthio-pyrrolidin,
cis- und trans-3-Amino-4-ethylthio-pyrrolidin,

Die Umsetzung von (II) mit (III) wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet oder auf das Verdünnungsmittel ganz verzichtet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 60°C und 150°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Carbonsäure (II) 1 bis 5 Mol, vorzugsweise 1 bis 3 Mol der Verbindung (III) ein.

Die bei der Umsetzung von (II) mit (III) entstehende Zwischenverbindung (IV) braucht nicht in Substanz Isoliert zu werden. Die hydrolytische Abspaltung der Oxoverbindung (V) aus der Zwischenverbindung (IV) gelingt häufig bereits bei der Aufarbeitung in Gegenwart von Wasser bei Raumtemperatur. Sie läßt sich durch Erwärmen beschleunigen. Unter diesen Bedingungen isoliert man das Reaktionsprodukt der Formel (I) als Betain.

Vorteilhaft ist es, nach Isolierung der Zwischenverbindung (IV) die Abspaltung der Schutzgruppe in Gegenwart äquivalenter oder überschüssiger Mengen einer Säure vorzunehmen, wobei das Reaktionsprodukt (I) in Form des Salzes dieser Säure isoliert werden kann. Als Säuren kommen beispielsweise Salzsäure, Schwefelsäure, Essigsäure, Trifluoressigsäure, Milchsäure, Glykolsäure, Bernsteinsäure, Zitronensäure, Weinsäure, Methansulfonsäure, p-Toluolsulfonsäure, Nicotinsäure, Galakturonsäure, Gluconsäure, Embonsäure, Glutaminsäure oder Asparaginsäure in Frage.

Die abgespaltene Oxoverbindung (V) läßt sich durch Extraktion mit einem Lösungsmittel wie z.B. Dichlormethan, Diethylether, Toluol, Essigsäureethylester oder tert.-Butyl-methyl-ether auf einfache Weise aus dem Reaktionsansatz zurückisolieren und erneut in die Reaktion einschleusen, was zu einer weiteren Vereinfachung und Verbilligung der Umsetzung führt.

Von großem Vorteil ist die Möglichkeit der schonenden Abspaltung in einem Zweiphasen-System aus wäßrig-verdünnter Säure und einem mit Wasser nicht mischbaren Extraktionsmittel, welches die Oxoverbindung (V) löst und so aus der Reaktionslösung entfernt, für Chinoloncarbonsäuren mit empfindlichen Resten.

So läßt sich beispielsweise 8-Chlor-1-cyclopropyl-6-fluor-1,4-dihydro-7-[3-(3-nitrobenzylidenamino)-1-pyrrolidinyl]-4-oxo-3-chinolincarbonsäure mit 3n-Salzsäure innerhalb 15 Minuten glatt deblockieren, ohne daß eine nennenswerte 8-Deschlorierung beobachtet wird. Dagegen erfolgt bei der Deblockierung von 7-(3-tert.-Butoxycarbonylamino-1-pyrrolidinyl)-3-chlor-1-cyclopropyl-6-fluor-1,4-dihydro-4-oxo-3-chinolincarbonsäure mit 3n-Salzsäure unter diesen Bedingungen selbst nach 30 Minuten nur zu etwa 10 % Abspaltung der Schutzgruppe, wobei das Spaltprodukt bereits durch 7-(3-Amino-1-pyrrolidinyl)-1-cyclopropyl-6-fluor-1,4-dihydro-4-oxo-3-chinolincarbonsäure (8-Deschlorderivat) verunreinigt ist.

Nach dem erfindungsgemäßen Verfahren lassen sich außer den in den Beispielen aufgeführten Wirkstoffen folgende Verbindungen herstellen, die als Racemate oder als enantiomerenreine Stoffe in Form der Salze (z.B. als Hydrochlorid, Sulfat, Methansulfonat, p-Toluolsulfonat oder Acetat) vorliegen können:

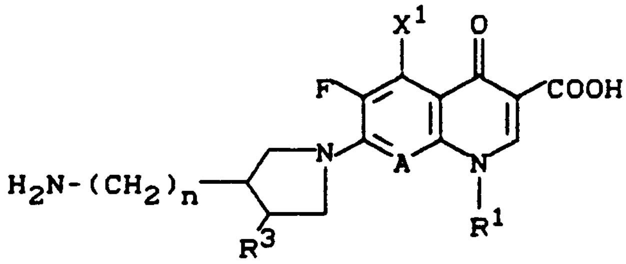

| $R^1$ | $R^3$ | $X^1$ | A | n |
|---|---|---|---|---|
| 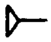 | H | H | N | 0 |
| 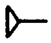 | H | H | N | 1 |
| 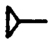 | $CH_3O$ | H | N | 0 |
| 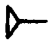 | $CH_3S$ | H | N | 0 |

(Fortsetzung)

| $R^1$ | $R^3$ | $X^1$ | A | n |
|---|---|---|---|---|
| 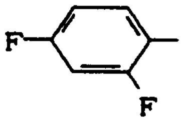 | H | H | N | 0 |
| 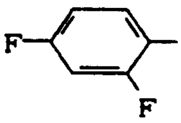 | $CH_3O$ | H | N | 0 |
| 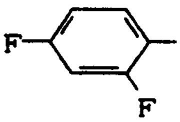 | $CH_3S$ | H | N | 0 |
| $(CH_3)_3C$- | H | H | N | 0 |
| $(CH_3)_3C$ | $CH_3O$ | H | N | 0 |
| $(CH_3)_3C$ | $CH_3S$ | H | N | 0 |
| 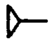 | H | $NH_2$ | CF | 0 |
| 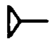 | H | $CH_3$ | CF | 0 |
| 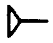 | H | $CH_3$ | CH | 0 |
| 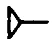 | H | $NH_2$ | CF | 1 |
| 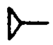 | H | $NH_2$ | CH | 0 |
| 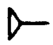 | H | $CH_3$ | CF | 1 |
| 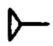 | H | F | CF | 0 |
| 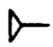 | $CH_3$ | H | CH | 0 |
| 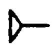 | $CH_3$ | H | CF | 0 |
| 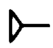 | $CH_3$ | H | CCl | 0 |
| 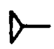 | $CH_3$ | H | N | 0 |

10-(3-Amino-1-pyrrolidinyl)-9-fluor-2,3-dihydro-3-methyl-7-oxo-7H-pyrido[1,2,3-de][1,4]benzoxacin-6-carbonsäure, 10-(3(S)-Amino-1-pyrrolidinyl)-9-fluor-2,3-dihydro-3(S)-methyl-7-oxo-7H-pyrido[1,2,3-de][1,4]benzoxacin-6-carbonsäure.

Die nachfolgenden Beispiele belegen die Erfindung:

Beispiel A

3-(2,2-Dimethyl-propyliden-amino)-pyrrolidin

1. 34,5 g (0,4 mol) 3-Aminopyrrolidin werden in 100 ml Toluol vorgelegt und 34,5 g (0,3 mol) 75 %iger Pivalaldehyd (Präparat der Firma Riedel-de-Haen) in 100 ml Toluol bei 20°C unter Wasserbadkühlung zugetropft. Es wird 2 Stunden bei Raumtemperatur nachgerührt, dann wird die wäßrige Phase im Scheidetrichter abgetrennt (4,6 ml) und die Toluollösung 1 Stunde über Natriumsulfat getrocknet. Das Toluol wird am Rotationsverdampfer abgetrennt und der Rückstand mit einer kleinen Vigreuxkolonne destilliert. Es werden 46,2 g 3-(2,2-Dimethylpropyliden-amino)-pyrrolidin vom Siedepunkt 61°C/10 mbar mit einer gaschromatographisch bestimmten Reinheit von 98 % erhalten, entsprechend einer Ausbeute von 74 % bzw. einer Selektivität von 99 % (Pivalaldehyd).
2. 8,6 g (0,1 mol) 3-Aminopyrrolidin werden vorgelegt und unter Wasserbadkühlung bei 20°C bis 30°C 8,8 g (0,1 mol) 97 %iger Pivalaldehyd (Präparat der Firma Aldrich) zugetropft. Es wird 1 Stunde bei Raumtemperatur nachgerührt und anschließend destilliert. Ausbeute: 13,1 g (85 % der Theorie), Siedepunkt: 60 - 63°C/10 mbar; Gehalt: 99 %.
3. 12,1 g (0,1 mol) 71 %iger Pivalaldehyd werden vorgelegt und bei 20°C bis 30°C unter Wasserbadkühlung 8,6 g (0,1 mol) 3-Aminopyrrolidin zugetropft. Es wird 1 Stunde bei Raumtemperatur nachgerührt und anschließend destilliert. Ausbeute: 13,5 g (85 % der Theorie), Siedepunkt: 63°C - 65°C/10 bis 12 mbar; Gehalt: 97 %.

Beispiele A 4-9

8,6 g (0,1 mol) 3-Aminopyrrolidin werden gegebenenfalls in 50 ml Lösungsmittel vorgelegt und Pivalaldehyd bei 20°C bis 30°C unter Wasserbadkühlung zugetropft. Es wird 1 Stunde bei Raumtemperatur nachgerührt, gegebenenfalls eine wäßrige Phase abgetrennt, das Lösungsmittel am Rotationsverdampfer entfernt und das Rohprodukt destilliert.

| Beispiel A | Pivalaldehyd [mol] | Lösungsmittel | 3-(2,2-Dimethyl-propylidenamino)-pyrrolidin | | |
|---|---|---|---|---|---|
| | | | Ausbeute bzw. Selektivität | | |
| | | | Gehalt [%] | [g] | [%] |
| 4 | 0,1 | THF | 98 | 12,5 | 79 |
| 5 | 0,1 | - | 97 | 13,0 | 82 |
| 6 | 0,09 | - | 94 | 13,9 | 94 |
| 7 | 0,12 | - | 96 | 13,5 | 84 |
| 8 | 0,09 | $CH_3OH$ | 95 | 13,1 | 90 |
| 9 | 0,09 | $CH_3$-O-C($CH_3$)$_3$ | 95 | 13,7 | 94 |

10. 36,8 g (0,138 mol) 1-Benzyl-3-benzylamino-pyrrolidin werden in 160 ml Methanol über 5 g 5 %igem Palladium auf Aktivkohle 10 Stunden bei 130°C und 100 bar Wasserstoffdruck hydriert. Der Katalysator wird abfiltriert und das Filtrat unter Rühren mit 16,6 g (0,138 mol) 71 %igem Pivalaldehyd versetzt, worauf die Temperatur von 20°C auf 28°C steigt. Es wird 30 Minuten nachgerührt, Methanol am Rotationsverdampfer entfernt und der Rückstand destilliert. Es werden 12,7 g 3-(2,2-Dimethyl-propyliden-amino)pyrrolidin vom Siedepunkt: 61°C/10 mbar erhalten mit einer gaschromatographisch bestimmten Reinheit von 97 %, entsprechend einer Ausbeute von 58 % der Theorie.

Beispiel B

3-(3,5-Difluorbenzyliden-amino)-pyrrolidin

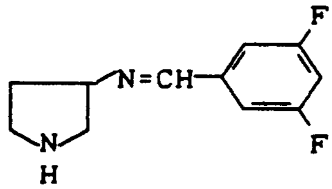

1,72 g (20 mmol) 3-Amino-pyrrolidin werden in 20 ml Methanol vorgelegt und unter Wasserbadkühlung bei 20°C 2,84 g (20 mmol) 3,5-Difluorbenzaldehyd zugetropft. Es wird eine Stunde bei Raumtemperatur nachgerührt, am Rotationsverdampfer im Vakuum eingeengt und der Rückstand (4,5 g) destilliert. Es werden 0,9 g eines Öls, Siedepunkt: 80°C - 85°C/0,04 mbar, erhalten, welches sich bei der Lagerung bei Raumtemperatur innerhalb von 4 Wochen zu einer hochviskosen Masse zersetzt.

Beispiel C

3-[(2,2-Dimethylpropylidenamino)-methyl]-pyrrolidin

2,0 g (20 mmol) 3-Aminomethyl-pyrrolidin werden in 10 ml Methanol vorgelegt und bei Raumtemperatur 1,8 g (22 mmol) 97 %iger Pivalaldehyd zugetropft, worauf die Innentemperatur auf 30°C steigt. Es wird 1 Stunde nachgerührt, danach eingeengt und der Rückstand über eine Vigreux-Kolonne destilliert.

Ausbeute: 2,8 g (80 % der Theorie) 97 %iges Produkt;
Siedepunkt: 84 - 88°C/11 bis 13 mbar.

Beispiel D

R-3-(2,2-Dimethyl-propylidenamino)-pyrrolidin

Analog Beispiel A-1 setzt man mit R-3-Aminopyrrolidin um und erhält R-3-(2,2-Dimethyl-propylidenamino)pyrrolidin vom Siedepunkt 62°C/8 mbar;
$\alpha_D^{20}$ = +36,3° (c = 1,002, $CHCl_3$).

Beispiel E

S-3-(2,2-Dimethyl-propylidenamino)-pyrrolidin

Analog Beispiel A-1 setzt man mit S-3-Aminopyrrolidin um und erhält S-3-(2,2-Dimethyl-propylidenamino)pyrrolidin vom Siedepunkt 61°C/10 mbar;
$\alpha_D^{20}$ = -36,5° (c = 1,0, $CHCl_3$).

Beispiel F

a) 1-Benzoyl-trans-3-amino-4-methylthio-pyrrolidin

41,5 g (0,24 mol) 1-Benzoyl-2,5-dihydropyrrol werden in 240 ml Dichlormethan vorgelegt und bei 0°C 24,8 g (0,3 mol) Methansulfonsäurechlorid zugetropft. Es wird 16 Stunden bei Raumtemperatur nachgerührt, dann wird das Lösungsmittel bei 8 mbar abgezogen und der Rückstand in 240 ml THF aufgenommen. Nach der Zugabe von 65 g 25%iger Ammoniaklösung wird im Autoklaven 10 Stunden auf 80°C erhitzt. Dann wird auf die 5-fache Menge Wasser

gegossen, mit Soda pH 10-11 eingestellt, mit Dichlormethan extrahiert und der Extrakt nach dem Trocknen über Natriumsulfat eingeengt. Das Rohprodukt (50 g) wird am Kieselgel chromatographiert (Laufmittel zunächst

Ethanol: Essigsäureethylester 1:3, dann Ethanol; RF-Wert: 0,34 (Ethanol).
Ausbeute: 33,5 g (59% der Theorie).

b) trans-3-Amino-4-methylthio-pyrrolidin

23,6 g (0,1 mol) 1-Benzoyl-trans-3-amino-4-methylthiopyrrolidin werden mit 80 ml 5 n Natronlauge gerührt, bis eine homogene Lösung entstanden ist (2 Stunden). Dann wird mit Natriumsulfat gesättigt und im Perforator mit tert.-Butyl-methyl-ether extrahiert. Der Extrakt wird über Natriumsulfat getrocknet, filtriert, eingeengt und der Rückstand destilliert.

Ausbeute: 10,5 g (79% der Theorie), Siedepunkt: 108 - 110°C / 11 mbar.

c) trans-3-(2,2-Dimethyl-propylidenamino)-4-methylthio-pyrrolidin

1,32 g (10 mmol) trans-3-Amino-4-methylthio-pyrrolidin werden in 5 ml Methanol vorgelegt und 1,23 ml (11 mmol) 97%iger Pivalaldehyd zugetropft, worauf die Temperatur von 25° auf 36°C steigt. Es wird noch 30 min. ohne Kühlung nachgerührt, dann wird der Ansatz eingeengt und der Rückstand destilliert. Ausbeute 1,50 g (75% der Theorie),

Siedepunkt: 63°C / 0,07 mbar.

Beispiel 1

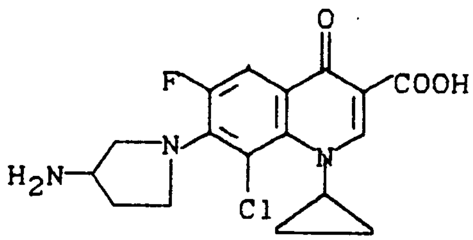

3 g (10 mmol) 8-Chlor-1-cyclopropyl-6,7-difluor-1,4-dihydro-4-oxo-3-chinolincarbonsäure werden in einer Mischung aus 20 ml Acetonitril und 10 ml Dimethylformamid mit 2,2 g (20 mmol) 1,4-Diazabicyclo[2.2.2]octan und 1,7 g (11 mmol) 3-(2,2-Dimethylpropylidenamino)pyrrolidin 1 Stunde unter Rückfluß erhitzt. Die Mischung wird im Vakuum eingeengt, der Rückstand mit Wasser verrührt, der ungelöste Rückstand abgesaugt, mit Wasser gewaschen und bei 70°C im Vakuum getrocknet. Das erhaltene Produkt wird aus einer Mischung von 35 ml Glykolmonomethylether und 5 ml Dimethylformamid umkristallisiert und mit Ethanol gewaschen.

Ausbeute: 3,2 g (87,6 % der Theorie) 7-(3-Amino-1-pyrrolidinyl)-8-chlor-1-cyclopropyl-6-fluor-1,4-dihydro-4-oxo-3-chinolincarbonsäure;
Schmelzpunkt: 254 - 256°C (unter Zersetzung);
Gehalt: 100 %ig (nach HPLC).

Beispiel 2

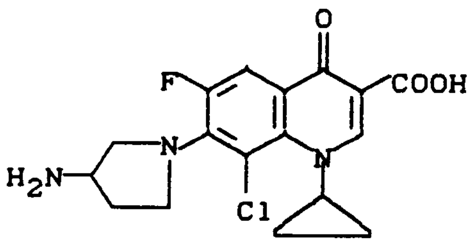

a) Man rührt eine Mischung aus 0,86 g (10 mmol) 3-Aminopyrrolidin, 1,8 g Molekularsieb (4 Å) und 0,95 g (11 mmol) Pivalaldehyd in 10 ml Acetonitril unter Feuchtigkeitsausschluß 1 Stunde bei Raumtemperatur, dekantiert die Lösung vom Molekularsieb ab, wäscht mit 10 ml Acetonitril nach und versetzt mit 10 ml Dimethylformamid. Danach wird mit 3 g (10 mmol) 8-Chlor-1-cyclopropyl-6,7-difluor-1,4-dihydro-3-chinolincarbonsäure und 2,2 g (20 mmol) 1,4-Diazabicyclo[2.2.2]octan versetzt und 1 Stunde unter Rückfluß erhitzt. Die Mischung wird eingedampft, mit Wasser verrührt (pH 7), der Niederschlag abgesaugt, mit Wasser gewaschen und getrocknet.

Ausbeute: 3,2 g (87,6 % der Theorie) 7-(3-Amino-1-pyrrolidinyl)-8-chlor-1-cyclopropyl-6-fluor-1,4-dihydro-4-oxo-3-chinolincarbonsäure; nach Umkristallisation aus Dimethylformamid erhält man 2,7 g des Produktes vom Schmelzpunkt 235°C (unter Zersetzung); Gehalt: 98,9 % (nach HPLC).

b) Zu einer Lösung von 0,86 g (10 mmol) 3-Aminopyrrolidin in 10 ml Acetonitril werden bei Raumtemperatur innerhalb von 5 Minuten 0,95 g (11 mmol) Pivalaldehyd zugetropft, wobei die Temperatur von 22,5°C auf 27,5°C ansteigt. Man läßt 1 Stunde nachrühren und stellt durch gaschromatographische Reaktionskontrolle einen praktisch vollständigen Umsatz zu 3-(2,2-Dimethylpropylidenamino)-pyrrolidin fest. Danach wird mit 10 ml Acetonitril und 10 ml Dimethylformamid verdünnt, mit 3 g (10 mmol) 8-Chlor-1-cyclopropyl-6,7-difluor-1,4-dihydro-3-chinolincarbonsäure und 2,2 g (20 mmol) 1,4-Diazabicyclo[2.2.2]octan versetzt und 1 Stunde unter Rückfluß erhitzt. Die Aufarbeitung liefert das gleiche Ergebnis wie unter Beispiel 2 a) beschrieben.

Beispiel 3

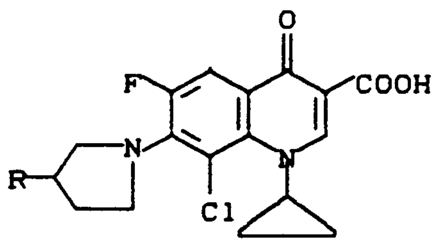

A: R = $(CH_3)_3C$-CH=N-
B: R = HCl x $H_2N$-

A) 0,86 g (10 mmol) 3-Aminopyrrolidin werden bei Raumtemperatur mit 1,1 g (12,8 mmol) Pivalinaldehyd versetzt,

wobei die Temperatur von 22°C auf 30°C steigt. Nach 1 Stunde wird mit 20 ml Acetonitril und 10 ml Dimethylformamid verdünnt, dann 3 g (10 mmol) 8-Chlor-1-cyclopropyl-6,7-difluor-1,4-dihydro-4-oxo-3-chinolincarbonsäure und 2,2 g (20 mmol) 1,4-Diazabicyclo[2.2.2]octan zugegeben und 1 Stunde unter Rückfluß erhitzt. Nach dem Abkühlen wird der Niederschlag abgesaugt, mit Acetonitril gewaschen und bei 100°C/12 mbar getrocknet.

Ausbeute: 3,25 g (75 % der Theorie) 8-Chlor-1-cyclopropyl-7-[3-(2,2-dimethylpropylidenamino)-1-pyrrolidinyl]-6-fluor-1,4-dihydro-4-oxo-3-chinolincarbonsäure vom Schmelzpunkt: 181°C bis 182°C.
FAB-Massenspektrum: m/e 434 (100 %, $M+H^{\oplus}$)
$^1$H-NMR-Spektrum ($CF_3COOD$): δ 1,45 s (9H), 1,25 m und 1,62 m (4H), 2,65 m und 2,80 m (2H), 4,2 m und 4,4 m (4H), 4,85 m (1H), 5,02 m (1H), 8,22 d (1H), 8,75 s (1H), 9,5 ppm s (1H).

B) 2,3 g (5,3 mmol) 8-Chlor-1-cyclopropyl-7-[3-(2,2-dimethylpropylidenamino)-1-pyrrolidinyl]-6-fluor-1,4-dihydro-4-oxo-3-chinolincarbonsäure werden bei Raumtemperatur in 15 ml Wasser suspendiert, mit 10 ml 1 n-Salzsäure bei etwa 60°C gelöst. Die Lösung wird filtriert, im Hochvakuum eingeengt und der Rückstand mit Ethanol verrührt. Der Niederschlag wird abgesaugt und im Vakuum getrocknet.

Ausbeute: 1,4 g (65,7 % der Theorie) 7-(3-Amino-1-pyrrolidinyl)-8-chlor-1-cyclopropyl-6-fluor-1,4-dihydro-4-oxo-3-chinolincarbonsäure-Hydrochlorid,
Schmelzpunkt: ab 280°C (unter Zersetzung).

Beispiel 4

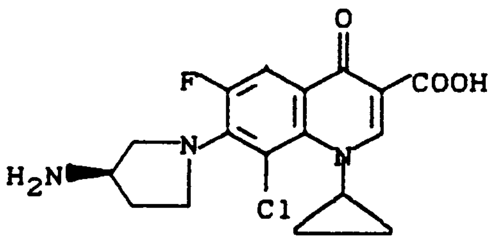

Analog Beispiel 1 wird mit R-3-(2,2-Dimethylpropylidenamino)-pyrrolidin zu 7-(R-3-Amino-1-pyrrolidinyl)-8-chlor-1-cyclopropyl-6-fluor-1,4-dihydro-4-oxo-3-chinolincarbonsäure vom Schmelzpunkt 252°C - 253°C (unter Zersetzung) (umkristallisiert aus Dimethylformamid) umgesetzt.

Beispiel 5

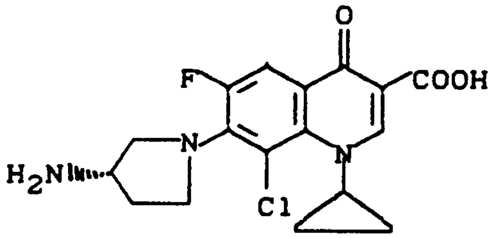

Analog Beispiel 1 wird mit S-3-(2,2-Dimethylpropylidenamino)-pyrrolidin zu 7-(S-3-Amino-1-pyrolidinyl)-8-chlor-1-cyclopropyl-6-fluor-1,4-dihydro-4-oxo-3-chinolincarbonsäure umgesetzt, Schmelzpunkt 253°C - 254°C (unter Zersetzung).

Beispiel 6

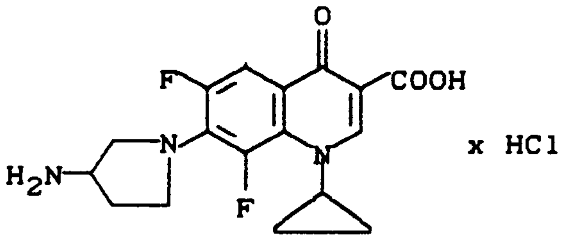

2,8 g (10 mMol) 1-Cyclopropyl-6,7,8-trifluor-1,4-dihydro-4-oxo-3-chinolincarbonsäure werden in einer Mischung aus 20 ml Acetonitril und 10 ml Dimethylformamid mit 2,2 g (20 mmol) 1,4-Diazabicyclo[2.2.2]octan und 1,7 g (11 mmol) 3-(2,2-Dimethylpropylidenamino)-pyrrolidin 1 Stunde unter Rückfluß erhitzt. Die Mischung wird eingeengt, der Rückstand mit Wasser verrührt, der Niederschlag abgesaugt und in 10 ml halbkonzentrierter Salzsäure gelöst, die Lösung filtriert und mit Ethanol das Hydrochlorid ausgefällt. Das Kristallisat wird abgesaugt, mit Ethanol gewaschen und bei 80°C/12 mbar getrocknet.

Ausbeute: 2,8 g (72,6 % der Theorie) 7-(3-Amino-1-pyrrolidinyl)-1-cyclopropyl-6,8-difluor-1,4-dihydro-4-oxo-3-chinolin-carbonsäure-Hydrochlorid;
Schmelzpunkt: 305°C - 306°C (unter Zersetzung).

Führt man die Umsetzung analog mit 3-(3,5-Difluorbenzylidenamino)-pyrrolidin aus, dann erhält man das gleiche Produkt in 75 % Ausbeute.

Beispiel 7

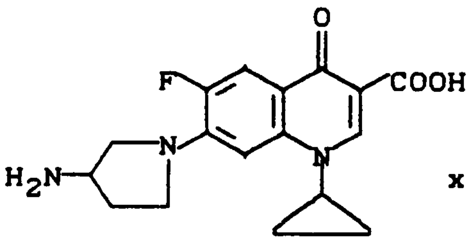

x HCl

Analog Beispiel 6 setzt man mit 1-Cyclopropyl-6,7-difluor-1,4-dihydro-4-oxo-3-chinolincarbonsäure um und erhält 7-(3-Amino-1-pyrrolidinyl)-1-cyclopropyl-6-fluor-1,4-dihydro-3-chinolincarbonsäure-Hydrochlorid vom Schmelzpunkt 301°C - 302°C (unter Zersetzung) in einer Ausbeute von 93,6 % der Theorie.

Beispiel 8

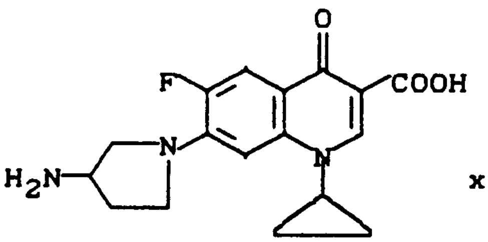

x HCl

Man versetzt 0,86 g (10 mmol) 3-Aminopyrrolidin mit 1,34 g (11 mmol) Salicylaldehyd, rührt etwa 10 Minuten bei Raumtemperatur und löst das zähe Reaktionsprodukt in einer Mischung aus 20 ml Acetonitril und 10 ml Dimethylformamid auf. Nach Zugabe von 2,2 g (20 mmol) 1,4-Diazabicyclo[2.2.2]octan und 2,65 g (10 mmol) 1-Cyclopropyl-6,7-difluor-1,4-dihydro-4-oxo-3-chinolincarbonsäure wird 1 Stunde unter Rückfluß erhitzt, abgekühlt, das auskristallisierte Reaktionsprodukt abgesaugt, mit Acetonitril gewaschen und in 30 ml halbkonzentrierter Salzsäure gelöst. Die Lösung wird eingeengt, der Rückstand mit Acetonitril verrührt, das Ungelöste in 30 ml Wasser in der Hitze gelöst, die Lösung filtriert und das Filtrat mit 100 ml Ethanol versetzt. Das ausfallende Hydrochlorid wird abgesaugt, mit Ethanol gewaschen und getrocknet.

Ausbeute: 2,6 g (70,7 % der Theorie) 7-(3-Amino-1-pyrrolidinyl)-1-cyclopropyl-6-fluor-1,4-dihydro-3-chinolincarbonsäure-Hydrochlorid;
Schmelzpunkt: 310°C - 317°C (unter Zersetzung).

Die Umsetzung verläuft in analoger Weise, wenn anstatt des Salicylaldehyds Benzaldehyd, 2-Chlorbenzaldehyd, 3-Chlorbenzaldehyd, 2,4-Dichlorbenzaldehyd, 2-Methylbenzaldehyd, 4-Methylbenzaldehyd, 4-Chlorbenzaldehyd, 4-Fluorbenzaldehyd oder 3,4-Difluorbenzaldehyd verwendet wird.

Beispiel 9

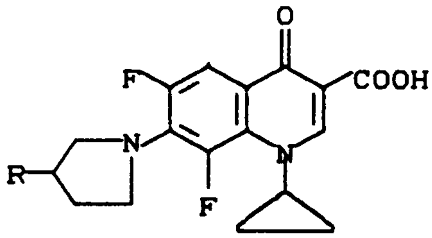

A: R =

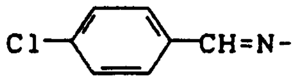

B: R = HCl x $H_2N$-

A) Eine Mischung aus 0,43 g (5 mmol) 3-Aminopyrrolidin und 0,79 g (5,5 mmol) 97 %igem 4-Chlor-benzaldehyd wird 1 Stunde ohne Lösungsmittel gerührt. Danach wird mit 10 ml Acetonitril und 5 ml Dimethylformamid verdünnt, mit 1,43 g (5 mmol) 1-Cyclopropyl-6,7,8-trifluor-1,4-dihydro-4-oxo-3-chinolincarbonsäure und 1,1 g 1,4-Diazabicyclo[2.2.2]octan versetzt und 1 Stunde unter Rückfluß erhitzt. Man kühlt ab, saugt den ausgefallenen Niederschlag ab, wäscht mit Acetonitril und trocknet bei 60°C im Vakuum.

Ausbeute: 2,22 g (94,2 % der Theorie) 7-[3-(4-Chlorbenzylidenamino)-1-pyrrolidinyl]-cyclopropyl-6,8-difluor-1,4-dihydro-4-oxo-3-chinolincarbonsäure;
Schmelzpunkt: 232°C - 237°C.
$^1$H-NMR ($CF_3COOD$): δ 7,75 d und 8,08 d (4H), 8,12 d (1H), 9,07 s (1H), 9,3 ppm s (1H)

B) 2,0 g (4,2 mmol) des Produkts aus Stufe A werden in 35 ml halbkonzentrierter Salzsäure heiß gelöst, die Lösung heiß filtriert, das Hydrochlorid nach dem Abkühlen abgesaugt, mit Ethanol gewaschen und getrocknet.

Ausbeute: 1,34 g (82 % der Theorie) 7-(3-Amino-1-pyrrolidinyl)-1-cyclopropyl-6,8-difluor-1,4-dihydro-3-chinolincarbonsäure-Hydrochlorid;
Schmelzpunkt: 302°C - 305°C (Zersetzung).

Beispiel 10

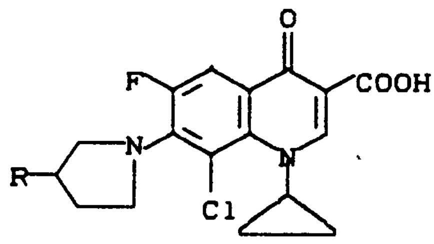

A: R =

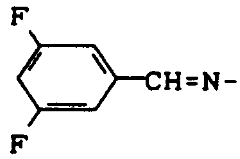

B: R = HCl x $H_2N$-

A) Analog Beispiel 9A) wird mit 3,5-Difluorbenzaldehyd umgesetzt und 1-Cyclopropyl-6,8-difluor-7-[3-(3,5-difluorbenzylidenamino)-1-pyrrolidinyl]-1,4-dihydro-4-oxo-3-chinolincarbonsäure vom Schmelzpunkt 225°C - 227°C (unter Zersetzung) isoliert;

Massenspektrum: m/e 473 ($M^{\oplus}$), 429 ($M^{\oplus}$-$CO_2$), 349, 346, 288;
Gehalt: 93 %ig.

B) Die Spaltung der Stufe A mit halbkonzentrierter Salzsäure verläuft analog Beispiel 9B) zu 7-(3-Amino-1-pyrrolidinyl)-1-cyclopropyl-6,8-difluor-1,4-dihydro-3-chinolincarbonsäure-Hydrochlorid,

Schmelzpunkt: 303°C - 306°C (unter Zersetzung).

Beispiel 11

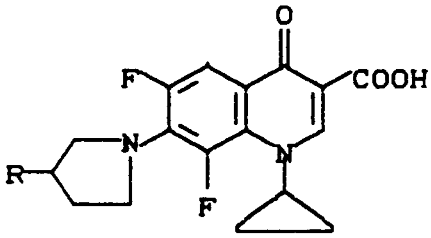

A: R =

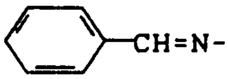

B: R = HCl x $H_2N$-

A) Analog Beispiel 9A) wird mit Benzaldehyd umgesetzt und 7-(3-Benzylidenamino-1-pyrrolidinyl)-1-cyclopropyl-6,8-difluor-1,4-dihydro-4-oxo-3-chinolincarbonsäure vom Schmelzpunkt 207°C - 208°C (Zersetzung) isoliert.

$^1$H-NMR ($d^6$-DMSO): δ 8,6 s (1H), 8,48 s (1H), 7,7 m (3H), 7,45 m (3H), 3,65-4,3 m (6H), 2,25 m und 2,05 m (2H), 1,2 ppm m (4H).

B) Die Spaltung der Stufe A) mit halbkonzentrierter Salzsäure erfolgt analog Beispiel 9B) zu 7-(3-Amino-1-pyrrolidinyl)-1-cyclopropyl-6,8-difluor-1,4-dihydro-3-chinolincarbonsäure-Hydrochlorid;

Schmelzpunkt: 300°C - 303°C (unter Zersetzung).

Beispiel 12

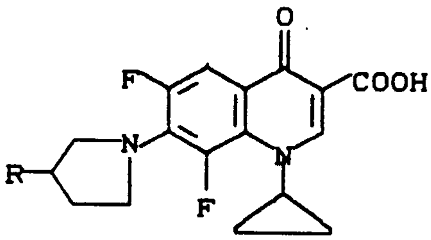

A: R = $(CH_3)_3C$-CH=N-
B: R = HCl x $H_2N$-

A) Analog Beispiel 9A) wird mit Pivalinaldehyd umgesetzt und 7-[3-(2,2-Dimethylpropylidenamino)-1-pyrrolidinyl]-6,8-difluor-1,4-dihydro-4-oxo-3-chinolincarbonsäure vom Schmelzpunkt 221°C - 222°C (unter Zersetzung) isoliert.

$^{1}$H-NMR-Spektrum ($CF_3COOD$): δ 1,46 s (9H), 1,44 m und 1,58 m (4H), 2,63 m und 2,75 m (2H), 4,2-4,55 m (5H), 5,0 m (1H), 8,1 dd (1H), 8,79 s (1H), 9,3 ppm s (1H).

B) Die Spaltung der Stufe A) mit halbkonzentrierter Salzsäure erfolgt analog Beispiel 9B) zu 7-(3-Amino-1-pyrrolidinyl)-1-cyclopropyl-6,8-difluor-1,4-dihydro-3-chinolincarbonsäure-Hydrochlorid;

Schmelzpunkt: 305°C - 308°C (unter Zersetzung).

<u>Beispiel 13</u>

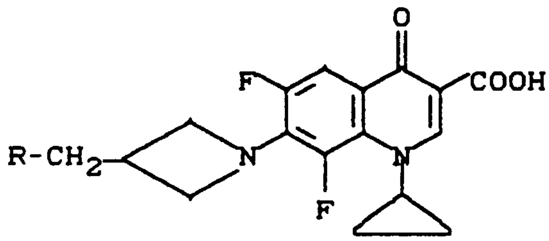

A: R = $(CH_3)_3C\text{-}CH{=}N\text{-}$
B: R = HCl x $H_2N$-

A) Analog Beispiel 9A) wird die Umsetzung mit Pivalinaldehyd und 3-Aminomethylazetidin durchgeführt und 1-Cyclopropyl-7-[3-(2,2-dimethylpropylidenaminomethyl)-1-azetidinyl]-6,8-difluor-1,4-dihydro-4-oxo-3-chinolincarbonsäure vom Schmelzpunkt 254°C - 256°C (unter Zersetzung) erhalten.

$^{1}$H-NMR (d6-DMSO): δ 8,57 s (1H), 7,68 s (1H), 7,65 ppm dd (1H).

B) Die Spaltung der Stufe A) mit halbkonzentrierter Salzsäure analog Beispiel 9B) liefert 7-(3-Aminomethyl-1-azetidinyl)-1-cyclopropyl-6,8-difluor-1,4-dihydro-3-chinolincarbonsäure-Hydrochlorid, das sich um etwa 200°C zersetzt.

FAB-Massenspektrum (positiv): m/e 386 $[(M+HCl+H^+)^{\oplus}]$
FAB-Massenspektrum (negativ): m/e 384 $[(M+Cl^-)^{\ominus}]$.

Beispiel 14

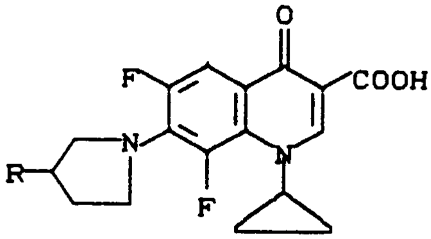

A: R =

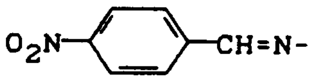

B: R = HCl x $H_2N$-

A) Zu einer Lösung von 0,86 g (10 mmol) 3-Aminopyrrolidin in 10 ml Acetonitril gibt man eine Lösung von 1,66 g (11 mmol) 4-Nitrobenzaldehyd in 10 ml Acetonitril und rührt 1 Stunde bei Raumtemperatur. Die Mischung wird mit 10 ml Dimethylformamid verdünnt, mit 2,2 g (20 mmol) 1,4-Diazabicyclo[2.2.2]octan und 2,83 g (10 mmol) 1-Cyclopropyl-6,7,8-trifluor-1,4-dihydro-4-oxo-3-chinolincarbonsäure versetzt und 1 Stunde unter Rückfluß erhitzt. Die Suspension wird eingeengt, der Rückstand mit 60 ml Acetonitril verrührt, der Niederschlag abgesaugt und bei 80°C im Vakuum getrocknet.

Ausbeute: 4,5 g (93 % der Theorie) 1-Cyclopropyl-6,8-difluor-1,4-dihydro-7-[3-(4-nitrobenzylidenamino)-1-pyrrolidinyl]-4-oxo-3-chinolincarbonsäure.
Schmelzpunkt: 227°C - 228°C (unter Zersetzung).

B) 4,3 g (8,9 mmol) des Produktes aus Stufe A werden in 40 ml halbkonzentrierter Salzsäure gelöst, die Lösung filtriert und eingeengt, der Rückstand mit 30 ml Ethanol verrührt, der ungelöste Niederschlag abgesaugt, mit Ethanol gewaschen und getrocknet.

Ausbeute: 3,14 g (91 % der Theorie) 7-(3-Amino-1-pyrrolidinyl)-1-cyclopropyl-6,8-difluor-1,4-dihydro-4-oxo-3-chinolincarbonsäure-Hydrochlorid;
Schmelzpunkt: 292°C - 294°C (unter Zersetzung).

Beispiele 15 bis 19

Analog Beispiel 14A) erhält man mit:

15A) 2-Nitrobenzaldehyd: 1-Cyclopropyl-6,8-difluor-1,4-dihydro-7-[3-(2-nitrobenzylidenamino)-1-pyrrolidinyl]-4-oxo-3-chinolincarbonsäure,
Schmelzpunkt: 227°C - 228°C (unter Zersetzung).
16A) 3-Nitrobenzaldehyd: 1-Cyclopropyl-6,8-difluor-1,4-dihydro-7-[3-(3-nitrobenzylidenamino)-1-pyrrolidinyl]-4-oxo-3-chinolincarbonsäure,
Schmelzpunkt: 264°C - 266°C (unter Zersetzung).
17A) 2,4-Dichlorbenzaldehyd: 1-Cyclopropyl-7-[3-(2,4-dichlorbenzylidenamino)-1-pyrrolidinyl]-6,8-difluor-1,4-dihydro-4-oxo-3-chinolincarbonsäure,

Schmelzpunkt: 209°C - 213°C (unter Zersetzung).

18A) 4-Methylbenzaldehyd: 1-Cyclopropyl-6,8-difluor-1,4-dihydro-7-[3-(4-methylbenzylidenamino)-1-pyrrolidinyl]-4-oxo-3-chinolincarbonsäure,

19A) 4-Methoxybenzaldehyd: 1-Cyclopropyl-6,8-difluor-1,4-dihydro-7-[3-(4-methoxybenzylidenamino)-1-pyrrolidinyl]-4-oxo-3-chinolincarbonsäure.

Die Verbindungen der Beispiele 15A) bis 19A) lassen sich analog Beispiel 14B) zu 7-(3-Amino-1-pyrrolidinyl)-1-cyclopropyl-6,8-difluor-1,4-dihydro-4-oxo-3-chinolincarbonsäure-Hydrochlorid umsetzen.

<u>Beispiel 20</u>

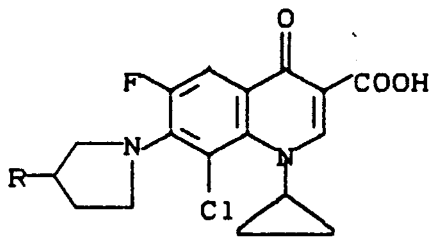

A: R =

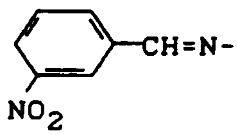

B: R = HCl x $H_2N$-

A) Eine Lösung von 34,4 g (0,4 mol) 3-Aminopyrrolidin in 400 ml Acetonitril wird mit einer Lösung von 66,4 g (0,44 mol) 3-Nitrobenzaldehyd in 400 ml Acetonitril versetzt und die Mischung 1 Stunde bei Raumtemperatur gerührt. Danach wird mit 400 ml Dimethylformamid verdünnt, mit 88 g (0,79 Mol) 1,4-Diazabicyclo[2.2.2]octan und 120 g (0,4 mol) 8-Chlor-1-cyclopropyl-6,7-difluor-1,4-dihydro-4-oxo-3-chinolincarbonsäure versetzt und 1 Stunde unter Rückfluß erhitzt (etwa 91°C bis 92°C). Die erhaltene Suspension wird im Eisbad abgekühlt, der ausgefallene Niederschlag abgesaugt, mit Acetonitril gewaschen und getrocknet.

Ausbeute: 168,5 g (84,5 % der Theorie) 8-Chlor-1-cyclopropyl-6-fluor-1,4-dihydro-7-[3-(3-nitrobenzylidenamino)-1-pyrrolidinyl]-4-oxo-3-chinolincarbonsäure;
Schmelzpunkt: 204°C - 207°C (unter Zersetzung).

B) 120 g (0,24 mol) des Produkts aus Stufe A) werden in einer Mischung aus 1,8 l Dichlormethan und 2,4 l 3 n-Salzsäure 15 Minuten unter leichtem Rückfluß erwärmt (Innentemperatur 38°C). Die Mischung wird im Eisbad abgekühlt, das ausgefallene Produkt abgesaugt, gut mit Acetonitril gewaschen und im Vakuum bei 25°C über KOH getrocknet.

Ausbeute: 82,7 g (85,5 % der Theorie) 7-(3-Amino-1-pyrrolidinyl)-8-chlor-1-cyclopropyl-6-fluor-1,4-dihydro-4-oxo-3-chinolincarbonsäure-Hydrochlorid;
Schmelzpunkt: 279°C - 281°C (unter Zersetzung)

| $C_{17}H_{17}ClFN_3O_3xHCl$ (402): | | | | |
|---|---|---|---|---|
| berechnet: | C 50,7 | H 4,5 | N 10,4 | Cl 17,7 |
| gefunden: | C 50,4 | H 4,6 | N 10,4 | Cl 17,4 |

Das Produkt enthält 0,01 % 7-(3-Amino-1-pyrrolidinyl)-1-cyclopropyl-6-fluor-1,4-dihydro-4-oxo-3-chinolincarbonsäure-Hydrochlorid (8-Deschlorderivat).

Durch Einengen der Dichlormethan-Phase kann der 3-Nitrobenzaldehyd in praktisch quantitativer Ausbeute zurückgewonnen werden.

Beispiele 21 bis 29

Analog Beispiel 20A) erhält man mit:

21A) 2-Nitrobenzaldehyd: 8-Chlor-1-cyclopropyl-6-fluor-1,4-dihydro-7-[3-(2-nitrobenzylidenamino)-1-pyrrolidinyl]-4-oxo-3-chinolincarbonsäure, Schmelzpunkt: 234-237°C (unter Zersetzung).
22A) 4-Nitrobenzaldehyd: 8-Chlor-1-cyclopropyl-6-fluor-1,4-dihydro-7-[3-(4-nitrobenzylidenamino)-1-pyrrolidinyl)-4-oxo-3-chinolincarbonsäure, Schmelzpunkt: 207-210°C (unter Zersetzung);
23A) 2,4-Dichlor-benzaldehyd: 8-Chlor-1-cyclopropyl-6-fluor-1,4-dihydro-7-[3-(2,4-dichlorbenzylidenamino)-1-pyrrolidinyl)-4-oxo-3-chinolincarbonsäure,
Schmelzpunkt: 198-202°C(unter Zersetzung).
24A) 2,6-Dichlor-benzaldehyd: 8-Chlor-7-[3-(2,6-dichlorbenzylidenamino)-1-pyrrolidinyl]-1-cyclopropyl-6-fluor-1,4-dihydro-4-oxo-3-chinolincarbonsäure,
25A) 4-Chlor-3-nitro-benzaldehyd: 8-Chlor-7-[3-(4-chlor-3-nitro-benzylidenamino-1-pyrrolidinyl]-1-cyclopropyl-6-fluor-1,4-dihydro-4-oxo-3-chinolincarbonsäure,
26A) 2-Methylbenzaldehyd: 8-Chlor-1-cyclopropenyl-6-fluor-1,4-dihydro-7-[3-(2-methylbenzylidenamino)-1-pyrrolidinyl] 1-4-oxo-3-chnolincarbonsäure,
27A) 4-Methylbenzaldehyd: 8-Chlor-1-cyclopropyl-6-fluor-1,4-dihydro-7-[3-(4-methylbenzlyidenamino)-1-pyrrolidinyl]-4-oxo-3-chinolincarbonsäure,
28A) 4-Methoxybenzaldhyd: 8-Chlor-1-cyclopropyl-6-fluor-1,4-dihydro-7-[3-(4-methoxybenzlyidenamino)-1-pyrrolidinyl)-4-oxo-3-chinolincarbonsäure,
29A) 4-Hydroxybenzaldehyd: 8-Chlor-1-cyclopropyl-6-fluor-1,4-dihydro-7-[3-(4-hydroxybenzylidenamino)-1-pyrrolidinyl]-4-oxo-3-chinolincarbonsäure.

Die Abspaltung der substituierten Benzyliden-Schutzgruppen erfolgt unter denselben Bedingungen wie in Beispiel 20B zu 7-(3-Amino-1-pyrrolidinyl)-8-chlor-1-cyclopropyl-6-fluor-1,4-dihydro-4-oxo-3-chinolincarbonsäure-Hydrochlorid.

Beispiel 30

A. 1 g (2 mmol) des Produkts aus Beispiel 20A werden in 30 ml Dichlormethan mit einer Lösung von 520 mg (3 mmol) p-Toluolsulfonsäure in 30 ml Wasser versetzt und 15 Minunten unter Rückfluß erhitzt. Nach dem Abkühlen wird abgesaugt, mit Ethanol gewaschen und getrocknet.

Ausbeute: 0,9 g (84 % der Theorie) 7-(3-Amino-1-pyrrolidinyl)-8-chlor-1-cyclopropyl-6-fluor-1,4-dihydro-4-oxo-3-chinolincarbonsäure-p-Toluolsulfonat, Schmelzpunkt 222-225°C (unter Zersetzung).

B. 1 g (2 mmol) des Produkts aus Beispiel 20A werden in 30 ml Dichlormethan mit einer Losung von 1,8 g Schwefelsäure in 30 ml Wasser 1 Stunde unter Rückfluß erhitzt. Nach dem Abkühlen wird abgesaugt, mit Ethanol gewaschen und getrocknet.

Ausbeute: 0,4 g (48 % der Theorie) 7-(3-Amino-1-pyrrolidinyl)-8-chlor-1-cyclopropyl-6-fluor-1,4-dihydro-4-oxo-3-chinolincarbonsäure-Sulfat,
Schmelzpunkt: 210-215°C (unter Zersetzung).

Beispiel 31

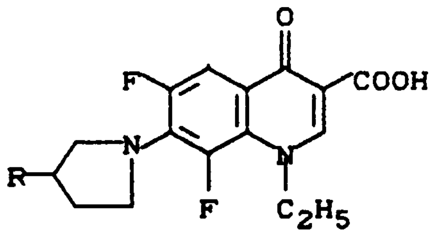

A. R = $(CH_3)_3C-CH=N-$
B. R = HCl X $H_2N-$

A. 2,7 g (10 mmol) 1-Ethyl-6,7,8-trifluor-1,4-dihydro-4-oxo-3-chinolincarbonsäure werden in einer Mischung aus 40 ml Acetonitril und 20 ml Dimethylformamid mit 2,2 g (20 mmol) 1,4-Diazabicyclo[2,2,2]octan und 1,7 g (11 mmol) 3-(2,2-Dimethylpropylidenamino)-pyrrolidin 1 Stunde unter Rückfluß erhitzt. Nach dem Abkühlen wird der ausgefallene Niederschlag abgesaugt, mit Acetonitril gewaschen und getrocknet.

Ausbeute: 3,57 g (88 % der Theorie) 7-[3-(2,2-Dimethylpropyliden-amino)-1-pyrrolidinyl]-1-ethyl-6,8-difluor-1,4-dihydro-4-oxo-3-chinolincarbonsäure, Schmelzpunkt: 250-252°C (unter Zersetzung).

B. 3,5 g (8,6 mmol) des Produkts aus Stufe A wird in eine Mischung aus 40 ml 4n-Salzsäure und 30 ml Dichlormethan eingetragen und 15 Minuten unter Rückfluß erhitzt. Nach dem Abkühlen wird abgesaugt, mit Ethanol gewaschen und getrocknet.

Ausbeute: 2,8 g (87 % der Theorie) 7-(3-Amino-1-pyrrolidinyl)-1-ethyl-6,8-difluor-1,4-dihydro-4-oxo-3-chinolincarbonsäure-Hydrochlorid, Schmelzpunkt: 288-290°C (unter Zersetzung).

Beispiel 32

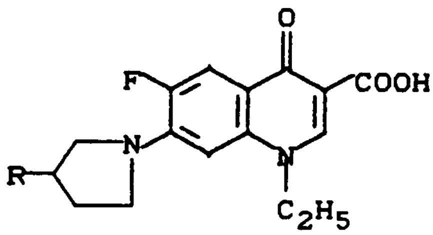

A. R = $(CH_3)_3C-CH=N-$
B. R = HCl X $H_2N-$

Analog Beispiel 31 erhält man mit 1-Ethyl-6,7-difluor-1,4-dihydro-4-oxo-3-chinoloncarbonsäure:

A. 7-[3-(2,2-Dimethylpropylidenamino)-1-pyrrolidinyl]-1-ethyl-6-fluor-1,4-dihydro-4-oxo-3-chinolincarbonsäure, Schmelzpunkt: 262-268°C (unter Zersetzung):

B. 7-(3-Amino-1-pyrrolidinyl)-1-ethyl-6-fluor-1,4-dihydro-4-oxo-3-chinolincarbonsäure-Hydrochlorid, Schmelzpunkt: 290-292°C (unter Zersetzung).

Beispiel 33

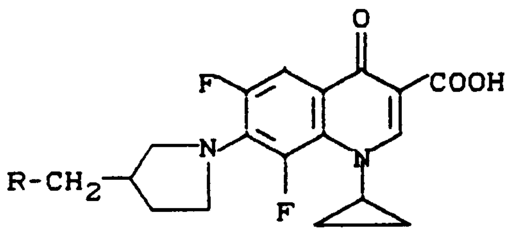

A. R = $(CH_3)_3C$-CH=N-

B. R =

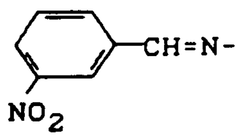

C. R = HCl x $H_2N$-

A. 1,42 g (5 mmol) 1-Cyclopropyl-6,7,8-trifluor-1,4-dichlor-4-oxo-3-chinolincarbonsäure werden in 20 ml Acetonitril und 10 ml Dimethylformamid mit 1,1 g (10 mmol) 1,4-Diazabicyclo[2.2.2]octan und 920 mg (5,5 mmol) 3-[(2,2-Dimethylpropylidenamino)methyl]-pyrrolidin 1 Stunde unter Rückfluß erhitzt. Der ausgefallene Niederschlag wird abgesaugt, mit Acetontril gewaschen und getrocknet.

Ausbeute: 1,87 g (87 % der Theorie) 1-Cyclopropyl-7-[3-(2,2-dimethylpropylidenamino-methyl)-1-pyrrolidinyl]-6,8-difluor-1,4-dihydro-4-oxo-3-chinolincarbonsäure, Schmelzpunkt 204-206°C (unter Zersetzung).

B. Man versetzt eine Lösung von 1,0 g (10 mmol) 3-Aminomethyl-pyrrolidin in 10 ml Acetonitril mit einer Lösung von 1,66 g (11 mmol) 3-Nitrobenzaldehyd in 10 ml Acetonitril, wobei die Temperatur der Mischung von 21°C auf 24°C steigt, und läßt 1 Stunde bei Raumtemperatur nachrühren. Anschließend wird die Mischung mit 10 ml Dimethylforamid verdünnt, mit 2,2 g (20 mmol) 1,4-Diazabicyclo[2.2.2]octan und 2,83 g (10 mmol) 1-Cyclopropyl-6,7,8-trifluor-1,4-dihydro-4-oxo-3-chinolincarbonsäure versetzt und 1 Stunde unter Rückfluß erhitzt. Der Niederschlag wird abgesaugt, mit Acetonitril gewaschen und getrocknet.

Ausbeute: 4,7 g (95 % der Theorie) 1-Cyclopropyl-6,8-difluor-1,4-dihydro-7-[3-(3-nitrobenzylidenaminomethyl)-1-pyrrolidinyl]-4-oxo-3-chinolincarbonsäure, Schmelzpunkt: 184-186°C (unter Zersetzung).

C. 2,7 g (5,4 mmol) des Produkts aus Beispiel 33B wird in einer Mischung aus 40 ml Dichlormethan und 40 ml 3n-Salzsäure wahrend 75 Minuten unter Rückfluß erhitzt. Nach dem Abkühlen wird der Niederschlag abgesaugt, mit Ethanol gewaschen und getrocknet.

Ausbeute: 1,35 g (62 % der Theorie) 7-(3-Aminomethyl-1-pyrrolidinyl)-1-cyclopropyl-6,8-difluor-1,4-dihydro-4-oxo-3-chinolincarbonsäure-Hydrochlorid, Schmelzpunkt: 290-295°C (unter Zersetzung).

Die Spaltung des Produkts aus Beispiel 33A mit Salzsäure liefert dasselbe Ergebnis.

Beispiel 34

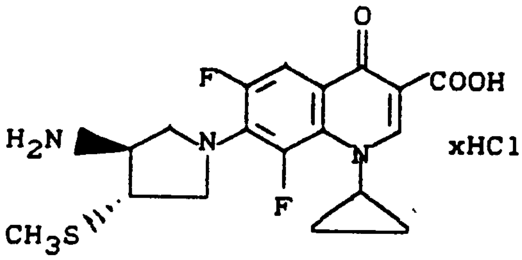

Analog Beispiel 33 A wird mit trans-3-(2,2-Dimethylpropylidenamino)-4-methylthio-pyrrolidin zu 1-Cyclopropyl-7-[trans-3-(2,2-dimethylpropylidenamino-4-methylthio)-1-pyrrolidinyl]-6,8-difluor-1,4-dihydro-4-oxo-3-chinolincarbonsäure umgesetzt und diese analog Beispiel 32 C zu 7-(trans-3-Amino-4-methylthio-1-pyrrolidinyl)-1-cyclopropyl-6,8-difluor-1,4-dihydro-4-oxo-3-chinolincarbonsäure-Hydrochlorid gespalten, Schmelzpunkt: 255-257°C (unter Zersetzung).

Beispiel 35

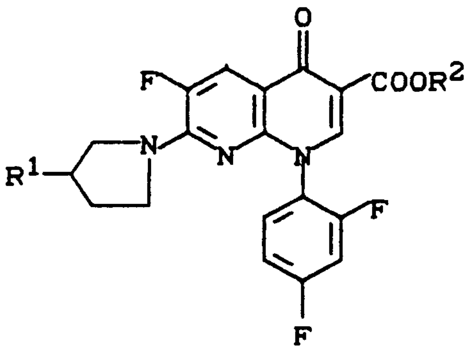

A. $R^1 = (CH_3)_3C\text{-}CH{=}N\text{-}$ , $R^2 = C_2H_5$
B. $R^1 = HCl \times H_2N\text{-}$ , $R^2 = H$

A. 0,86 g (10 mmol) 3-Aminopyrrolidin werden bei Raumtemperatur mit 1,1 g (12,8 mmol) Pivalinaldehyd versetzt und die Mischung nach 1 Stunde mit 20 ml Acetonitril und 10 ml Dimethylformaldehyd verdünnt. Man versetzt mit 1,1 g (10 mmol) 1,4-Diazabicyclo[2.2.2]octan und 3,8 g (10 mmol) 7-Chlor-1-(2,4-difluorphenyl)-6-fluor-1,4-dihydro-4-oxo-1,8-naphthyridin-3-carbonsäureethylester und rührt bei Raumtemperatur über Nacht. Der angefallene Niederschlag wird abgesaugt, mit Wasser gewaschen und im Vakuum bei 100°C getrocknet.

Ausbeute: 4,25 g (85 % der Theorie) 1-(2,4-Difluorphenyl)-7-[3-(2,2-dimethylpropylidenamino)-1-pyrrolidinyl]-6-fluor-1,4-dihydro-4-oxo-1,8-naphthyridin-3-carbonsäureethylester vom Schmelzpunkt 197-198°C (unter Zersetzung).

$^1$H-NMR ($CDCl_3$) : δ 1,05s (9H), 1,4t (3H), 1,9m und 2,05m (2H), um 3,5-3,7 breites m (6H), 4,4q (2H), 7,05m (2H, 7,4m (1H), 8,05d (1H), 8,35 ppm s (1H).

B. 1,5 g (3 mmol) des Produkts aus Stufe A werden in einer Mischung aus 15 ml Essigsäure und 12 ml halbkonzentrierter Salzsäure 6 Stunden unter Rückfluß erhitzt. Die Mischung wird im Vakuum eingeengt, der Rückstand mit Dichlormethan gewaschen und aus Glykolmonomethylether umkristallisiert.

Ausbeute: 0,9 g (68 % der Theorie) 7-(3-Amino-1-pyrrolidinyl)-1-(2,4-difluorphenyl)-6-fluor-1,4-dihydro-4-oxo-1,8-naphthyridin-3-carbonsäure-Hydrochlorid vom Schmelzpunkt 275-283°C (unter Zersetzung).

**Patentansprüche**

**1.** Verfahren zur Herstellung von Verbindungen der Formel (I), die als Enantiomeren- oder Diastereomerengemische oder in enantiomeren- oder diastereomerenreiner Form vorliegen können,

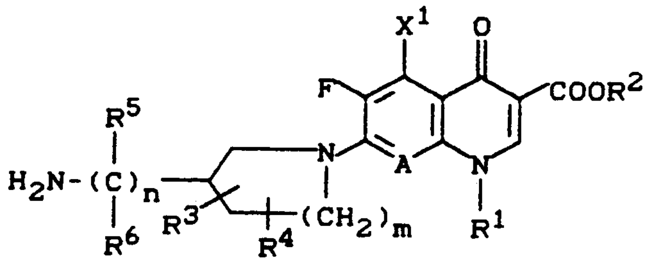

(I)

und in welcher

| | |
|---|---|
| $X^1$ | für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen oder Alkyl mit 1 bis 4 Kohlenstoffatomen, |
| $R^1$ | für geradkettiges oder verzweigtes Alkyl mit 1 bis 5 Kohlenstoffatomen, das gegebenenfalls durch 1 bis 3 Halogenatome substituiert sein kann, sowie Alkenyl mit 2 bis 4 Kohlenstoffatomen, Cycloalkyl mit 3 bis 6 Kohlenstoffatomen, 2-Hydroxyethyl, Methoxy, Amino, Methylamino, Ethylamino, Dimethylamino oder gegebenenfalls durch 1 oder 2 Fluoratome substituiertes Phenyl, |
| $R^2$ | für Wasserstoff, Alkyl mit 1 bis 4 Kohlenstoffatomen oder (5-Methyl-2-oxo-1,3-dioxol-4-yl)-methyl, |
| $R^3$ | für Wasserstoff, $C_1$-$C_3$-Alkyl, Hydroxy, $C_1$-$C_3$-Alkoxy, $C_1$-$C_3$-Alkylthio, Halogen, |
| $R^4$ | für Wasserstoff oder Methyl steht, |
| $R^5$ und $R^6$ | gleich oder verschieden sind und für Wasserstoff der Methyl stehen, |
| m | für 0, 1 oder 2, |
| n | für 0 oder 1 und |
| A | für N oder C-$R^7$ steht, worin |
| $R^7$ | für H, Halogen, Methyl, Cyano, Nitro, Hydroxy oder Methoxy steht oder auch gemeinsam mit $R^1$ eine Brücke der Struktur bilden kann, welche R- oder S-Konfiguration besitzen kann, |

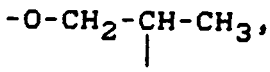, 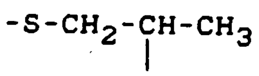 oder

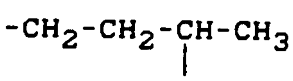

dadurch gekennzeichnet, daß man Verbindungen der Formel (II)

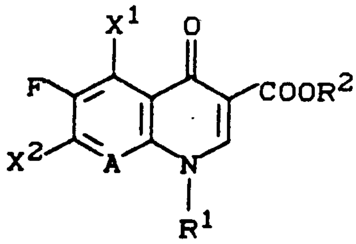

(II),

in welcher

$X^1$, $R^1$, $R^2$ und A die oben angegebene Bedeutung haben und
$X^2$ für Halogen steht,

mit Verbindungen der Formel (III),

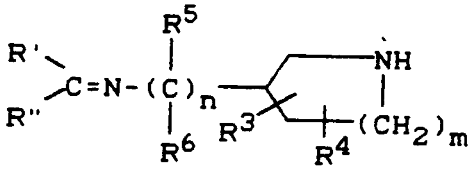

(III),

die in situ oder in Substanz durch Kondensation einer Oxoverbindung der Formel (V)

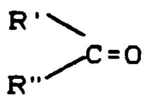

(V) ,

mit einer Aminoverbindung der Formel (VI)

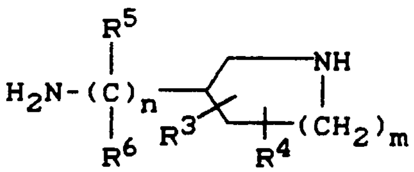

(VI)

erhalten werden können und in welchen

$R^3$, $R^4$, $R^5$, $R^6$, m und n die oben angegebene Bedeutung haben und

- R' für Wasserstoff, geradkettiges oder verzweigtes Alkyl mit 1 bis 10 Kohlenstoffatomen oder Phenyl, das gegebenenfalls durch $C_1$-$C_4$-Alkyl, $C_1$-$C_4$-Alkoxy, $C_1$-$C_2$-Alkylthio, Hydroxy, Nitro, Halogen, Carboxy, $C_1$-$C_4$-Alkoxycarbonyl, Cyano oder Phenyl ein- bis fünffach, gleich oder verschieden substituiert sein kann oder Naphthyl und
- R'' für Wasserstoff, geradkettiges oder verzweigtes Alkyl mit 1 bis 5 Kohlenstoffatomen oder Phenyl steht,

wobei R' und R'' auch gemeinsam mit dem Kohlenstoffatom, an welchem sie gebunden sind, einen gegebenenfalls durch Methyl oder Ethyl ein- oder mehrfach substituierten 5- oder 6-gliedrigen aliphatischen Ring bilden können, gegebenenfalls in Gegenwart eines säurebindenden Mittels zu Verbindungen der Formel (IV)

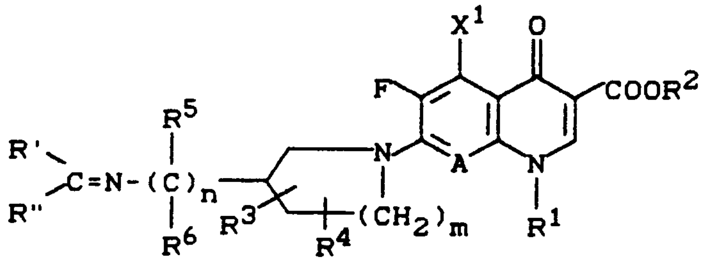

(IV),

in welcher
$X^1$, A, $R^1$, $R^2$, $R^3$, $R^4$, $R^5$, $R^6$, R', R'', m und n die oben angegebene Bedeutung haben, umsetzt und anschließend die Aminoschutzgruppe abspaltet.

**2.** Verfahren nach Anspruch 1, dadurch gekennzeichnet, daß man Verbindungen der Formel (II)

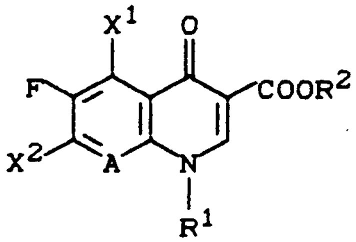

(II),

in welcher

- $X^1$ für Wasserstoff, Amino, Hydroxy, Alkoxy mit 1 bis 2 Kohlenstoffatomen, Fluor, Chlor, Brom, Alkyl mit 1 bis 3 Kohlenstoffatomen,
- $X^2$ für Fluor oder Chlor,
- $R^1$ für geradkettiges oder verzweigtes Alkyl mit 1 bis 4 Kohlenstoffatomen, das gegebenenfalls durch 1 bis 3 Fluoratome substituiert sein kann, sowie Alkenyl mit 2 bis 3 Kohlenstoffatomen, Cycloalkyl mit 3 oder 4 Kohlenstoffatomen, 2-Hydroxyethyl, 2-Fluorethyl, Methylamino, Ethylamino, Dimethylamino oder gegebenenfalls durch 1 oder 2 Fluoratome substituiertes Phenyl,
- $R^2$ für Wasserstoff, Alkyl mit 1 bis 3 Kohlenstoffatomen und
- A für N oder C-$R^7$ steht, worin
- $R^7$ für H, Fluor, Chlor, Brom, Methyl, Hydroxy oder Methoxy steht oder auch gemeinsam mit $R^1$ eine Brücke der Struktur

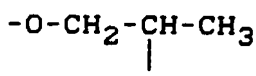

bilden kann,

sowie Verbindungen der Formel (III)

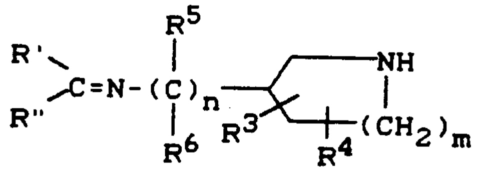

(III),

in welcher

- $R^3$ für Wasserstoff, Methyl, Ethyl, Hydroxy, Methoxy, Ethoxy, Methylthio, Ethylthio, Chlor oder Fluor,
- $R^4$ für Wasserstoff von Methyl,
- $R^5$ für Wasserstoff,
- $R^6$ für Wasserstoff,
- m für 0 oder 1,
- n für 0 oder 1,
- R' für Wasserstoff, geradkettiges oder verzweigtes Alkyl mit 1 bis 6 Kohlenstoffatomen oder Phenyl, das gegebenenfalls durch Methyl, Methoxy, Ethoxy, Hydroxy, Nitro, Fluor, Chlor, Carboxy, $C_1$-$C_3$-Alkoxycarbonyl oder Cyano ein-bis fünffach gleich oder verschieden substituiert sein kann, und
- R'' für Wasserstoff, geradkettiges oder verzweigtes Alkyl mit 1 bis 4 Kohlenstoffatomen oder Phenyl steht, wobei R' und R'' auch gemeinsam mit dem Kohlenstoffatom, an welchem sie gebunden sind, einen 5- oder 6-gliedrigen aliphatischen Ring können, verwendet.

**3.** Verfahren nach Anspruch 1, dadurch gekennzeichnet, daß man Verbindungen der Formel (II)

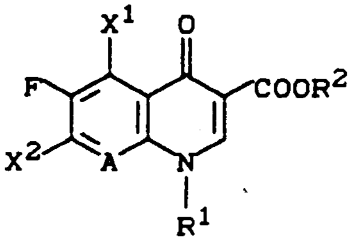

(II),

in welcher

- $X^1$ für Wasserstoff, Amino, Methoxy, Fluor, Chlor oder Methyl,
- $X^2$ für Fluor oder Chlor,
- $R^1$ für Methyl, Ethyl, tert.-Butyl, Vinyl, Cyclopropyl, 2-Fluorethyl, 4-Fluorphenyl oder 2,4-Difluorphenyl,
- $R^2$ für Wasserstoff, Methyl oder Ethyl,
- A für N oder C-$R^7$ steht, worin

  $R^7$ für Wasserstoff, Fluor, Chlor, Methyl oder Methoxy steht oder auch gemeinsam mit $R^1$ eine Brücke der Struktur

$$-O-CH_2-CH-CH_3$$

bilden kann,

sowie Verbindungen der Formel (III)

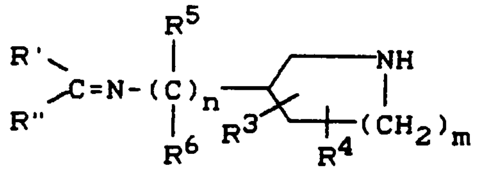

(III),

in welcher

- $R^3$ für Wasserstoff, Methyl, Hydroxy, Methoxy, Methylthio, Ethylthio oder Fluor,
- $R^4$ für Wasserstoff oder Methyl,
- $R^5$ für Wasserstoff,
- $R^6$ für Wasserstoff,
- m für 0 oder 1,
- n für 0 oder 1,
- R' für Wasserstoff, geradkettiges oder verzweigtes Alkyl mit 1 bis 5 Kohlenstoffatomen oder Phenyl, das gegebenenfalls durch Methyl, Methoxy, Hydroxy, Fluor, Chlor, Carboxy oder $C_1$-$C_2$-Alkoxycarbonyl ein- bis vierfach gleich oder verschieden substituiert sein kann, und
- R'' für Wasserstoff, geradkettiges oder verzweigtes Alkyl mit 1 bis 3 Kohlenstoffatomen oder Phenyl steht, wobei R' und R'' auch gemeinsam mit dem Kohlenstoffatom, an welchem sie gebunden sind, einen 5- oder 6-gliedrigen aliphatischen Ring können, verwendet.

**4.** Verbindungen der Formel (III)

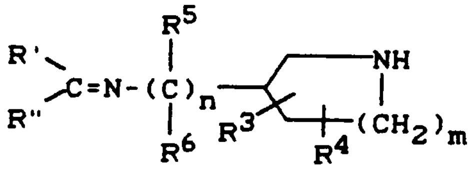

(III),

in der R', R'', $R^3$, $R^4$, $R^5$, $R^6$, n und m die in den Ansprüchen 1, 2 und 3 angegebene Bedeutung haben.

**5.** Verbindungen der Formel (IV)

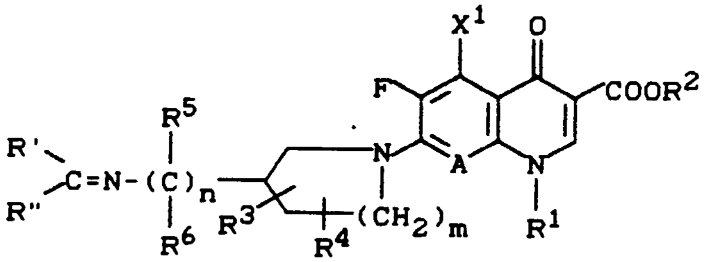

(IV) ,

in welcher

$X^1$, A, $R^1$, $R^2$, $R^3$, $R^4$, $R^5$, $R^6$, R', R'', m und n die in den Ansprüchen 1, 2 und 3 angegebene Bedeutung haben, ausgenommen Verbindungen, in denen gleichzeitig R'' für H, n für 0, m für 1, $R^3$ und $R^4$ für H, $X^1$ für H, A für CH, $R^2$ für H oder eine Carboxy schützende Gruppe, $R^1$ für $C_1$-$C_5$-Alkyl, $C_3$-$C_6$-Cycloalkyl gegebenenfalls durch 1 oder 2 Fluoratome substituiertes Phenyl und R' für Phenyl oder substituiertes Phenyl steht.

## Claims

1. Process for the preparation of compounds of formula (I) which can exist as enantiomer or diastereomer mixtures or in the form of pure enantiomers or diastereomers,

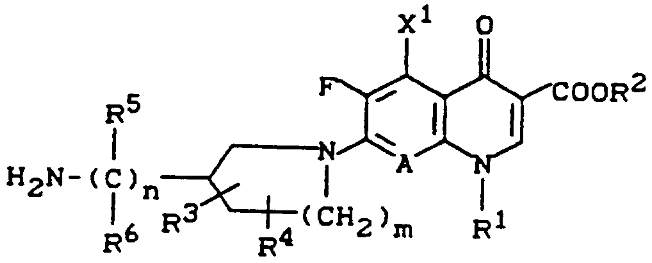

(I)

and in which

| | |
|---|---|
| $X^1$ | represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen or alkyl having 1 to 4 carbon atoms, |
| $R^1$ | represents straight-chain or branched alkyl having 1 to 5 carbon atoms which can optionally be substituted by 1 to 3 halogen atoms, and also alkenyl having 2 to 4 carbon atoms, cycloalkyl having 3 to 6 carbon atoms, 2-hydroxyethyl, methoxy, amino, methylamino, ethylamino, dimethylamino or phenyl which is optionally substituted by 1 or 2 fluorine atoms, |
| $R_2$ | represents hydrogen, alkyl having 1 to 4 carbon atoms or (5-methyl-2-oxo-1,3-dioxol-4-yl)-methyl, |
| $R^3$ | represents hydrogen, $C_1$-$C_3$-alkyl, hydroxyl, $C_1$-$C_3$-alkoxy, $C_1$-$C_3$-alkylthio, halogen, |
| $R^4$ | represents hydrogen or methyl, |
| $R^5$ and $R^6$ | are identical or different and represent hydrogen or methyl, |
| m | represents 0, 1 or 2, |
| n | represents 0 or 1 and |
| A | represents N or C-$R^7$, where |
| $R^7$ | represents H, halogen, methyl, cyano, nitro, hydroxyl or methoxy, or else together with $R^1$ can form a bridge having the structure |

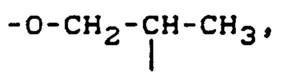

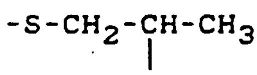

or

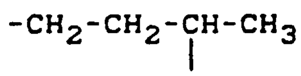

which can have the R- or S-configuration,

characterized in that compounds of the formula (II)

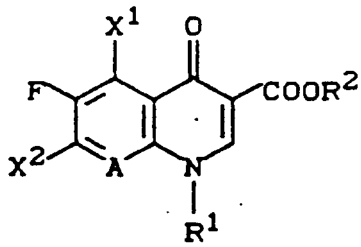

(II),

in which

$X^1$, $R^1$ $R^2$ and A have the abovementioned meaning and

$X^2$ represents halogen are reacted with compounds of the formula (III),

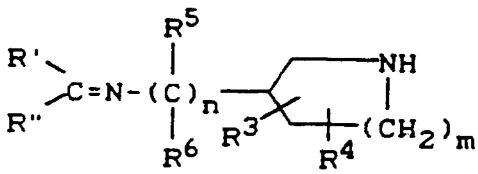

(III),

which can be obtained in situ or in substance by subjecting an oxo compound of the formula (V)

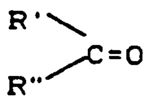

(V) ,

to a condensation reaction with an amino compound of the formula (VI)

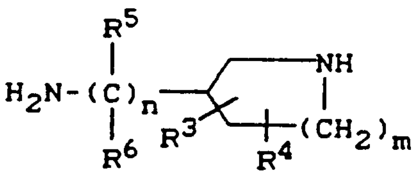

(VI)

and in which

$R^3$, $R^4$, $R^5$, $R^6$, m and n have the abovementioned meaning and

R' represents hydrogen, straight-chain or branched alkyl having 1 to 10 carbon atoms or phenyl which can optionally be mono- to pentasubstituted by identical or different substituents from the series consisting of $C_1$-$C_4$-alkyl, $C_1$-$C_4$-alkoxy, $C_1$-$C_2$-alkylthio, hydroxyl, nitro, halogen, carboxyl, $C_1$-$C_4$-alkoxy-carbonyl, cyano or phenyl, or represents naphthyl, and

R'' represents hydrogen, straight-chain or branched alkyl having 1 to 5 carbon atoms or phenyl, it also being possible for R' and R'' together with the carbon atom to which they are bonded to form a 5- or 6-membered aliphatic ring which is optionally mono- or polysubstituted by methyl or ethyl,

if appropriate in the presence of an acid binder, to give compounds of the formula (IV)

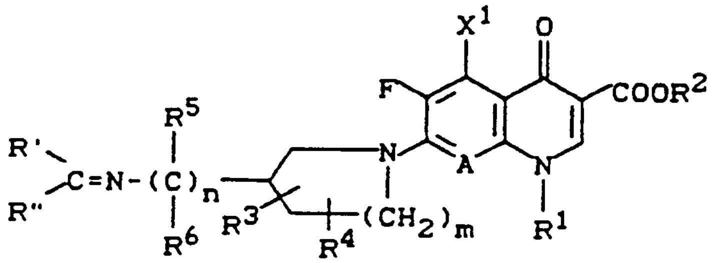

(IV),

in which

$X^1$, A, $R^1$, $R^2$, $R^3$, $R^4$, $R^5$, $R^6$, R', R", m and n have the abovementioned meaning, and the amino protective group is subsequently cleaved off.

**2.** Process according to Claim 1, characterized in that compounds of the formula (II)

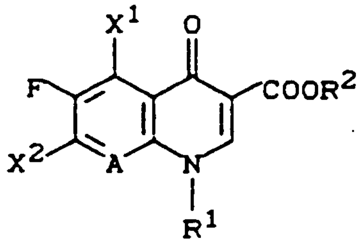

(II),

in which

$X^1$ represents hydrogen, amino, hydroxyl, alkoxy having 1 to 2 carbon atoms, fluorine, chlorine, bromine, alkyl having 1 to 3 carbon atoms,

$X^2$ represents fluorine or chlorine,

$R^1$ represents straight-chain or branched alkyl having 1 to 4 carbon atoms which can optionally be substituted by 1 to 3 fluorine atoms, and also alkenyl having 2 to 3 carbon atoms, cycloalkyl having 3 or 4 carbon atoms, 2-hydroxyethyl, 2-fluoroethyl, methylamino, ethylamino, dimethylamino or phenyl which is optionally substituted by 1 or 2 fluorine atoms,

$R^2$ represents hydrogen, alkyl having 1 to 3 carbon atoms and

A represents N or C-$R^7$, where

$R^7$ represents H, fluorine, chlorine, bromine, methyl, hydroxyl or methoxy or else together with $R^1$ can form a bridge having the structure

$$-O-CH_2-CH-CH_3,$$

and compounds of the formula (III)

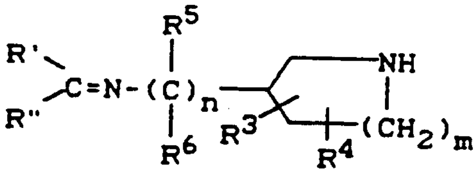

(III),

in which

- $R^3$ represents hydrogen, methyl, ethyl, hydroxyl, methoxy, ethoxy, methylthio, ethylthio, chlorine or fluorine,
- $R^4$ represents hydrogen or methyl,
- $R^5$ represents hydrogen,
- $R^6$ represents hydrogen,
- m represents 0 or 1,
- n represents 0 or 1,
- R' represents hydrogen, straight-chain or branched alkyl having 1 to 6 carbon atoms or phenyl which can optionally be mono- to pentasubstituted by identical or different substituents from the series consisting of methyl, methoxy, ethoxy, hydroxyl, nitro, fluorine, chlorine, carboxyl, $C_1$-$C_3$-alkoxycarbonyl or cyano, and
- R'' represents hydrogen, straight-chain or branched alkyl having 1 to 4 carbon atoms or phenyl, it also being possible for R' and R'' together with the carbon atom to which they are bonded to form a 5- or 6-membered aliphatic ring are used.

**3.** Process according to Claim 1, characterized in that compounds of the formula (II)

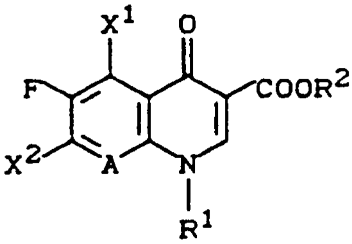

(II),

in which

- $X^1$ represents hydrogen, amino, methoxy, fluorine, chlorine or methyl,
- $X^2$ represents fluorine or chlorine,
- $R^1$ represents methyl, ethyl, tert-butyl, vinyl, cyclopropyl, 2-fluoroethyl, 4-fluorophenyl or 2,4-difluorophenyl,
- $R^2$ represents hydrogen, methyl or ethyl,

A represents N or C-$R^7$, where

$R^7$ represents hydrogen, fluorine, chlorine, methyl or methoxy or else together with $R^1$ can form a bridge having the structure

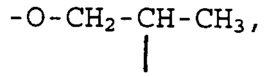

and compounds of the formula (III)

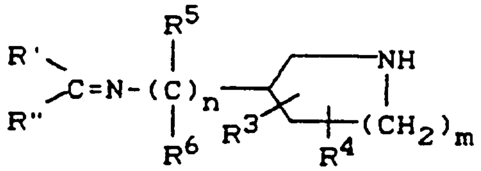

(III),

in which

$R^3$ represents hydrogen, methyl, hydroxyl, methoxy, methylthio, ethylthio or fluorine,

$R^4$ represents hydrogen or methyl,

$R^5$ represents hydrogen,

$R^6$ represents hydrogen,

m represents 0 or 1,

n represents 0 or 1,

R' represents hydrogen, straight-chain or branched alkyl having 1 to 5 carbon atoms or phenyl which can optionally be mono- to tetrasubstituted by identical or different substituents from the series consisting of methyl, methoxy, hydroxyl, fluorine, chlorine, carboxyl or $C_1$-$C_2$-alkoxycarbonyl and

R'' represents hydrogen, straight-chain or branched alkyl having 1 to 3 carbon atoms or phenyl, it also being possible for R' and R'' together with the carbon atom to which they are bonded to form a 5- or 6-membered aliphatic ring, are used.

**4.** Compounds of the formula (III)

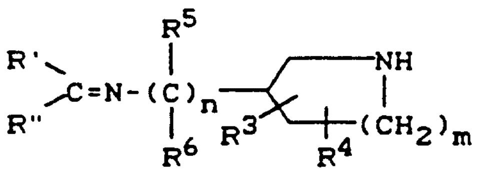

(III),

in which R', R'', $R^3$, $R^4$, $R^5$, $R^6$, n and m have the meaning given in Claims 1, 2 and 3.

**5.** Compounds of the formula (IV)

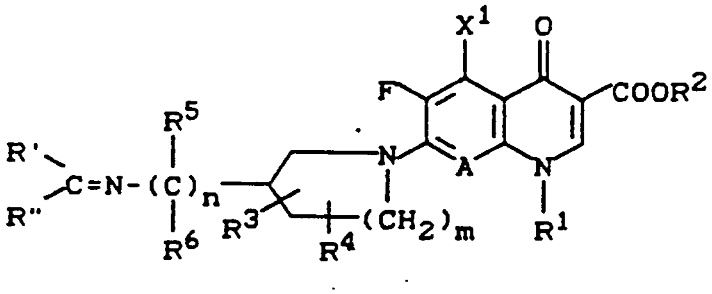

(IV) ,

in which

$X^1$, A, $R^1$, $R^2$, $R^3$, $R^4$, $R^5$, $R^6$, R', R", m and n have the meaning given in Claims 1, 2 and 3, with the exception of compounds in which simultaneously R" represents H, n represents 0, m represents 1, $R^3$ and $R^4$ represent H, $X^1$ represents H, A represents CH, $R^2$ represents H or a carboxyl-protecting group, $R^1$ represents $C_1$-$C_5$-alkyl, $C_3$-$C_6$-cycloalkyl, phenyl which is optionally substituted by 1 or 2 fluorine atoms, and R' represents phenyl or substituted phenyl.

## Revendications

**1.** Procédé pour la préparation de composés répondant à la formule (I) qui peuvent être présents sous la forme de mélanges d'énantiomères ou de diastéréoisomères ou sous la forme d'énantiomères ou de diastéréo-isomères à l'état pur,

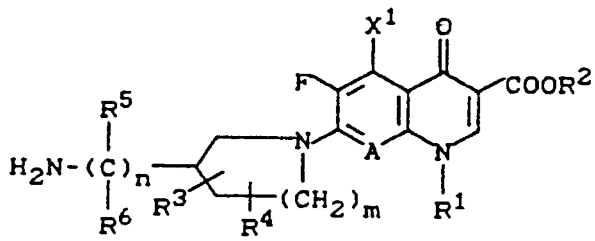

(I)

et dans laquelle

$X^1$ représente un atome d'hydrogène, un groupe amino, un groupe alkylamino contenant de 1 à 4 atomes de carbone, un groupe dialkylamino dans lequel chaque groupe alkyle contient de 1 à 3 atomes de carbone, un groupe hydroxyle, un groupe alcoxy contenant de 1 à 4 atomes de carbone, un groupe mercapto, un groupe alkylthio contenant de 1 à 4 atomes de carbone, un groupe arylthio, un atome d'halogène ou un groupe alkyle contenant de 1 à 4 atomes de carbone,

$R^1$ représente un groupe alkyle à chaîne droite ou ramifiée contenant de 1 à 5 atomes de carbone, qui le cas échéant peut porter de 1 à 3 substituants halogéno, identiques ou différents, ainsi qu'un groupe alcényle contenant de 2 à 4 atomes de carbone, un groupe cycloalkyle contenant de 3 à 6 atomes de carbone, un groupe 2-hydroxyéthyle, un groupe méthoxy, un groupe amino, un groupe méthylamino, un groupe éthylamino, un groupe diméthylamino ou encore un groupe phényle portant le cas échéant 1 ou 2 substituants fluoro,

$R^2$ représente un atome d'hydrogène, un groupe alkyle contenant de 1 à 4 atomes de carbone ou un groupe (5-méthyl-2-oxo-1,3-dioxol-4-yl)méthyle,

$R^3$ représente un atome d'hydrogène, un groupe alkyle en $C_1$-$C_3$, un groupe hydroxyle, un groupe alcoxy en $C_1$-$C_3$, un groupe alkyl(en $C_1$-$C_3$)thio, un atome d'halogène,

$R^4$ représente un atome d'hydrogène ou un groupe méthyle,

$R^5$ et $R^6$ sont identiques ou différents et représentent un atome d'hydrogène ou un groupe méthyle,

m représente 0, 1 ou 2,

n représente 0 ou 1, et

A représente un atome d'azote ou un groupe C-$R^7$, où

$R^7$ représente un atome d'hydrogène, un atome d'halogène, un groupe méthyle, un groupe cyano, un groupe nitro, un groupe hydroxyle ou un groupe méthoxy ou peut former, de manière conjointe avec $R^1$, un pont de structure

$$-O-CH_2-CH-CH_3, \quad -S-CH_2-CH-CH_3 \text{ ou}$$

$$-CH_2-CH_2-CH-CH_3$$

qui peut posséder une configuration R ou une configuration S,

caractérisé en ce qu'on fait réagir des composés répondant à la formule (II)

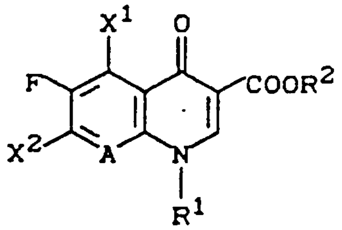

(II),

dans laquelle

$X^1$, $R^1$, $R^2$ et A ont la signification indiquée ci-dessus, et

$X^2$ représente un atome d'halogène,

avec des composés répondant à la formule (III)

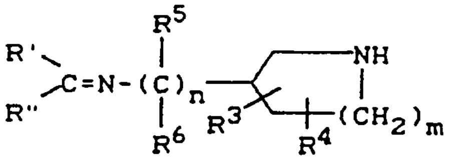

(III),

que l'on peut obtenir in situ ou en soi par condensation d'un composé oxo répondant à la formule (V)

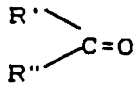 (V) ,

avec un composé amino répondant à la formule (VI)

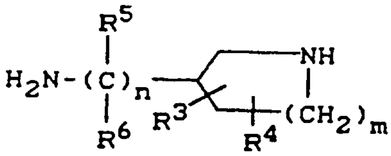 (VI)

et dans lesquelles $R^3$, $R^4$, $R^5$, $R^6$, m et n ont la signification indiquée ci-dessus, et

R' représente un atome d'hydrogène, un groupe alkyle à chaîne droite ou ramifiée contenant de 1 à 10 atomes de carbone ou encore un groupe phényle qui, le cas échéant, peut porter de 1 à 5 substituants identiques ou différents alkyle en $C_1$-$C_4$, alcoxy en $C_1$-$C_4$, alkyl(en $C_1$-$C_2$)thio, hydroxyle, nitro, halogéno, carboxyle, alcoxy(en $C_1$-$C_4$) carbonyle, cyano ou phényle, ou encore un groupe naphtyle et

R'' représente un atome d'hydrogène, un groupe alkyle à chaîne droite ou ramifiée contenant de 1 à 5 atomes de carbone ou un groupe phényle,

R' et R'' pouvant également former de manière conjointe avec l'atome de carbone auquel ils sont liés, un noyau aliphatique pentagonal ou hexagonal portant le cas échéant un ou plusieurs substituants méthyle ou éthyle,

le cas échéant en présence d'un agent neutralisant les acides, pour obtenir des composés répondant à la formule (IV)

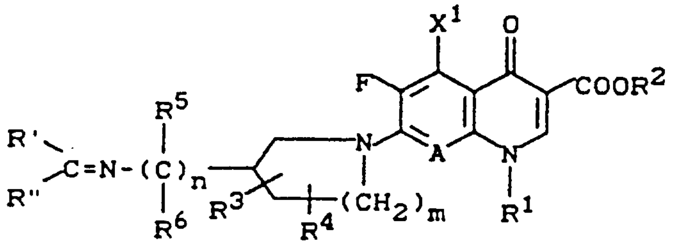 (IV),

dans laquelle

$X^1$, A, $R^1$, $R^2$, $R^3$, $R^4$, $R^5$, $R^6$, R', R'', m et n ont la signification indiquée ci-dessus, puis on élimine le groupe protecteur du groupe amino.

**2.** Procédé selon la revendication 1, caractérisé en ce qu'on utilise des composés répondant à la formule (II)

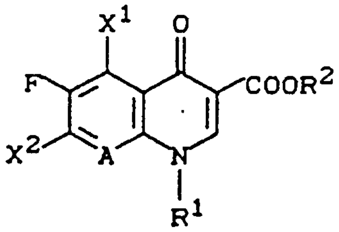

(II),

dans laquelle

$X^1$ représente un atome d'hydrogène, un groupe amino, un groupe hydroxyle, un groupe alcoxy contenant de 1 à 2 atomes de carbone, un atome de fluor, un atome de chlore, un atome de brome, un groupe alkyle contenant de 1 à 3 atomes de carbone,

$X^2$ représente un atome de fluor ou un atome de chlore,

$R^1$ représente un groupe alkyle à chaîne droite ou ramifiée contenant de 1 à 4 atomes de carbone, qui le cas échéant peut porter de 1 à 3 substituants fluoro, ainsi qu'un groupe alcényle contenant de 2 à 3 atomes de carbone, un groupe cycloalkyle contenant 3 ou 4 atomes de carbone, un groupe 2-hydroxyéthyle, un groupe 2-fluoréthyle, un groupe méthylamino, un groupe éthylamino, un groupe diméthylamino ou encore un groupe phényle portant le cas échéant 1 ou 2 substituants fluoro,

$R^2$ représente un atome d'hydrogène, un groupe alkyle contenant de 1 à 3 atomes de carbone, et

A représente un atome d'azote ou un groupe C-$R^7$ où

$R^7$ représente un atome d'hydrogène, un atome de fluor, un atome de chlore, un atome de brome, un groupe méthyle, un groupe hydroxyle ou un groupe méthoxy, ou encore peut également former, de manière conjointe avec $R^1$, un pont de structure

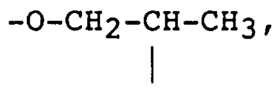

ainsi que des composés répondant à la formule (III)

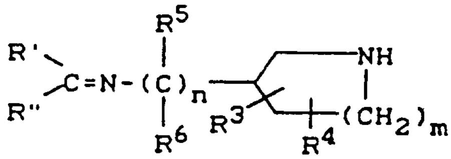

(III),

dans laquelle

$R^3$ représente un atome d'hydrogène, un groupe méthyle, un groupe éthyle, un groupe hydroxyle, un groupe

méthoxy, un groupe éthoxy, un groupe méthylthio, un groupe éthylthio, un atome de chlore ou un atome de fluor,

$R^4$ représente un atome d'hydrogène ou un groupe méthyle,

$R^5$ représente un atome d'hydrogène,

$R^6$ représente un atome d'hydrogène,

m représente 0 ou 1,

n représente 0 ou 1,

R' représente un atome d'hydrogène, un groupe alkyle à chaîne droite ou ramifiée contenant de 1 à 6 atomes de carbone ou encore un groupe phényle qui peut porter le cas échéant de 1 à 5 substituants identiques ou différents méthyle, méthoxy, éthoxy, hydroxyle, nitro, fluoro, chloro, carboxyle, alcoxy(en $C_1$-$C_3$) carbonyle ou cyano, et

R" représente un atome d'hydrogène, un groupe alkyle à chaîne droite ou ramifiée contenant de 1 à 4 atomes de carbone ou encore un groupe phényle,
R' et R" pouvant également former un noyau aliphatique pentagonal ou hexagonal de manière conjointe avec l'atome de carbone auquel ils sont liés.

**3.** Procédé selon la revendication 1, caractérisé en ce qu'on utilise des composés répondant à la formule (II)

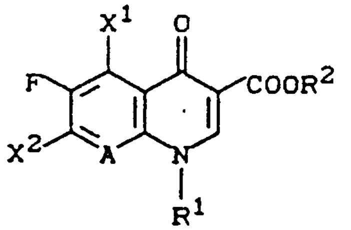

(II),

dans laquelle

$X^1$ représente un atome d'hydrogène, un groupe amino, un groupe méthoxy, un atome de fluor, un atome de chlore ou un groupe méthyle,

$X^2$ représente un atome de fluor ou un atome de chlore,

$R^1$ représente un groupe méthyle, un groupe éthyle, un groupe tert.-butyle, un groupe vinyle, un groupe cyclopropyle, un groupe 2-fluoréthyle, un groupe 4-fluorophényle ou un groupe 2,4-difluorophényle,

$R^2$ représente un atome d'hydrogène, un groupe méthyle ou un groupe éthyle,

A représente un atome d'azote ou un groupe C-$R^7$ où

$R^7$ représente un atome d'hydrogène, un atome de fluor, un atome de chlore, un groupe méthyle ou un groupe méthoxy, ou peut également former, de manière conjointe avec $R^1$, un pont de structure

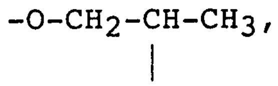

ainsi que des composés répondant à la formule (III)

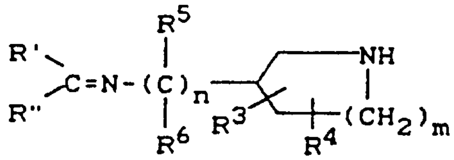

(III),

dans laquelle

- $R^3$ représente un atome d'hydrogène, un groupe méthyle, un groupe hydroxyle, un groupe méthoxy, un groupe méthylthio, un groupe éthylthio ou un atome de fluor,
- $R^4$ représente un atome d'hydrogène ou un groupe méthyle,
- $R^5$ représente un atome d'hydrogène,
- $R^6$ représente un atome d'hydrogène,
- m représente 0 ou 1,
- n représente 0 ou 1,
- R' représente un atome d'hydrogène, un groupe alkyle à chaîne droite ou ramifiée contenant de 1 à 5 atomes de carbone ou encore un groupe phényle qui, le cas échéant, peut porter de 1 à 4 substituants identiques ou différents méthyle, méthoxy, hydroxyle, fluoro, chloro, carboxyle ou alcoxy(en $C_1$-$C_2$)carbonyle, et
- R'' représente un atome d'hydrogène, un groupe alkyle à chaîne droite ou ramifiée contenant de 1 à 3 atomes de carbone ou encore un groupe phényle,
  R' et R'' pouvant également former un noyau aliphatique pentagonal ou hexagonal de manière conjointe avec l'atome de carbone auquel ils sont liés.

**4.** Composés répondant à la formule (III)

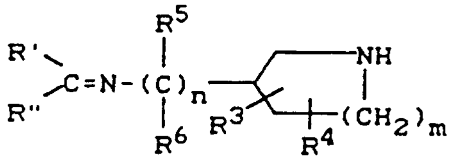

(III),

dans laquelle R', R'', $R^3$, $R^4$, $R^5$, $R^6$, n et m ont la signification indiquée dans les revendications 1, 2 et 3.

**5.** Composés répondant à la formule (IV)

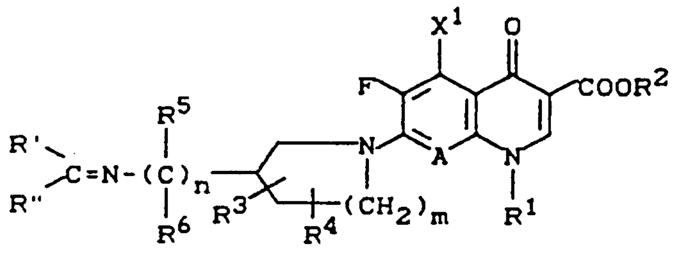

(IV),

dans laquelle

$X^1$, A, $R^1$, $R^2$, $R^3$, $R^4$, $R^5$, $R^6$, R', R", m et n ont la signification indiquée dans les revendications 1, 2 et 3, à l'exception des composés dans lesquels, de manière simultanée, R" représente un atome d'hydrogène, n représente 0, m représente 1, $R^3$ et $R^4$ représentent un atome d'hydrogène, $X^1$ représente un atome d'hydrogène, A représente un groupe CH, $R^2$ représente un atome d'hydrogène ou un groupe protecteur du groupe carboxyle, $R^1$ représente un groupe alkyle en $C_1$-$C_5$, un groupe cycloalkyle en $C_3$-$C_6$, un groupe phényle portant le cas échéant 1 ou 2 substituants fluoro et R' représente un groupe phényle ou un groupe phényle substitué.